(19) Europäisches Patentamt
European Patent Office
Office européen des brevets

(11) Publication number: **0 198 687 A2**

(12) **EUROPEAN PATENT APPLICATION**

(21) Application number: **86302707.4**

(22) Date of filing: **11.04.86**

(51) Int. Cl.⁴: **C 07 C 131/00**, A 01 N 37/16, A 01 N 47/06, A 01 N 47/24

(30) Priority: **12.04.85 US 722556**

(43) Date of publication of application: **22.10.86 Bulletin 86/43**

(84) Designated Contracting States: **AT BE CH DE FR GB IT LI LU NL SE**

(71) Applicant: **Rohm and Haas Company, Independence Mall West, Philadelphia, Pennsylvania 19105 (US)**

(72) Inventor: **Chi-Tung Hsu, Adam, 1686 Heebner Way, Lansdale, Pa. 19446 (US)**

(74) Representative: **Angell, David Whilton et al, Rohm and Haas Company Patent Department Chesterfield House Bloomsbury Way, London WC1A 2TP (GB)**

(54) **Alpha-polyhaloketoximes.**

(57) Alpha-polyhaloketoximes having the formula:

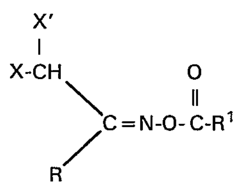

wherein

X and X' are the same or different halogen atoms;

R is methyl or halomethyl; and

$R^1$ is $(C_1-C_{12})$alkyl optionally substituted with one or more halogen, $(C_1-C_4)$alkoxy, cyano or nitro;

$(C_3-C_6)$cycloalkyl;

straight or branched chain $(C_1-C_4)$alkoxy optionally substituted with one or more halogen;

an amino group of the formula $NHR^2$ where $R^2$ is hydrogen, straight or branched chain $(C_1-C_4)$alkyl optionally substituted with one or more of the same or different halogen, $(C_3-C_6)$cycloalkyl, straight or branched chain $(C_2-C_6)$alkenyl, or aryl;

a thio group of the formula $SR^3$ where $R^3$ is straight or branched chain $(C_1-C_4)$alkyl optionally substituted with one or more of the same or different halogen, or aryl;

$(C_2-C_6)$alkenyl optionally substituted with one or more halogen;

aryl optionally substituted with one or more of the same of different halogen, cyano, nitro, $(C_1-C_4)$alkyl, $(C_1-C_4)$-alkoxy, or $(C_1-C_4)$haloalkyl,

a heterocyclic group;

phenalkyl where the alkyl moiety contains one to four carbon atoms and the phenyl ring is optionally substituted with one or more of the same or different halogen, cyano, nitro, $(C_1-C_4)$alkyl, $(C_1-C_4)$alkoxy, or $(C_1-C_4)$haloalkyl;

phenalkenyl where the alkenyl moiety contains two to five carbon atoms and the phenyl ring is optionally substituted with one or more of the same or different halogen, cyano, nitro, $(C_1-C_4)$alkyl, $(C_1-C_4)$alkoxy, or $(C_1-C_4)$haloalkyl;

phenoxy optionally substituted with one or more of the same or different halogen, cyano, nitro, $(C_1-C_4)$alkyl, $(C_1-C_4)$alkoxy, or $(C_1-C_4)$haloalkyl; or

benzyloxy where the phenyl ring is optionally substituted with one or more of the same or different halogen, cyano, nitro, $(C_1-C_4)$alkyl, $(C_1-C_4)$alkoxy, or $(C_1-C_4)$haloalkyl

are useful biocides, fungicides or pesticides.

# ALPHA-POLYHALOKETOXIMES

This invention is concerned with novel alpha-polyhaloketoximes which exhibit activity as biocides, fungicides and pesticides, with methods of making them, with compositions containing them and with methods of combating bacteria, fungi and insects using them.

Certain ketoxime derivatives have been shown to be effective agents for controlling insects, fungi and bacteria. The effectiveness of a given oxime derivative, however, cannot be predicted from an examination of the substituent groups. Closely related compounds may demonstrate quite different bacterial, fungal and pest controlling capabilities. In addition, various oxime derivatives may overlap or have complementary areas of activity or selectivity, and therefore, be useful in combination to control a variety of organisms upon application of a single composition.

The search for compounds having a combination of excellent activity and low toxicity is a continuing one. The alpha-polyhaloketoximes of this invention are believed to be less toxic than the known ketoxime derivatives while demonstrating useful activity.

The novel alpha-polyhaloketoximes (hereinafter referred to at times as "polyhaloketoximes") of this invention are represented by the formula

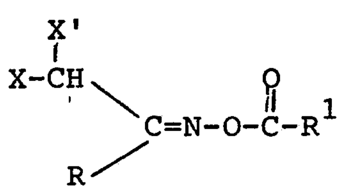

I

wherein

X and X' are the same or different halogen atoms selected from chloro, fluoro, bromo or iodo;

R is methyl or halomethyl;

$R^1$ is straight or branched chain $(C_1-C_{12})$-alkyl optionally substituted with one or more of the same or different halogen, $(C_1-C_4)$-alkoxy, cyano or nitro;

$(C_3-C_6)$-cycloalkyl;

straight or branched chain $(C_1-C_4)$-alkoxy optionally substituted with one or more halogen;

an amino group of the formula $NHR^2$ where $R^2$ is hydrogen or straight or branched chain $(C_1-C_4)$-alkyl optionally substituted with one or more of the same or different halogen, $(C_3-C_6)$-cycloalkyl, straight or branched chain $(C_2-C_6)$-alkenyl, or aryl;

a thio group of the formula $SR^3$ where $R^3$ is straight or branched chain $(C_1-C_4)$-alkyl optionally substituted with one or more of the same or different halogen, or aryl;

straight or branched chain $(C_2-C_6)$ alkenyl optionally substituted with one or more halogen;

aryl optionally substituted with one or more of the same or different halogen, cyano, nitro, $(C_1-C_4)$-alkyl, $(C_1-C_4)$-alkoxy, or $(C_1-C_4)$-haloalkyl;

a heterocyclic group;

phenalkyl where the alkyl moiety contains one to four carbon atoms and the phenyl ring is optionally substituted with one or more of the same or different halogen, cyano, nitro, $(C_1-C_4)$-alkyl, $(C_1-C_4)$-alkoxy, or $(C_1-C_4)$-haloalkyl;

phenalkenyl where the alkenyl moiety contains two to five carbon atoms and the phenyl ring is optionally substituted with one or more of the same or different halogen, cyano, nitro, $(C_1-C_4)$-alkyl, $(C_1-C_4)$-alkoxy, or $(C_1-C_4)$-haloalkyl;

phenoxy optionally substituted with one or more of the same or different halogen, cyano, nitro, $(C_1-C_4)$-alkyl, $(C_1-C_4)$-alkoxy, or $(C_1-C_4)$-haloalkyl; or

benzyloxy where the phenyl ring is optionally substituted with one or more of the same or different halogen, cyano, nitro, $(C_1-C_4)$-alkyl, $(C_1-C_4)$-alkoxy, or $(C_1-C_4)$-haloalkyl.

The term "halogen" includes chlorine, fluorine, bromine and iodine. The term "aryl" should be

understood as including groups such as benzene, naphthalene and the like having up to 10 nuclear carbon atoms. The term "halomethyl" should be understood as including mono-, di- and trihalomethyl groups such as chloromethyl, dichloromethyl, trifluoromethyl and dibromomethyl. The term "alkyl" should be understood as including groups containing the stated number of carbon atoms such as methyl, ethyl, propyl, butyl, hexyl, heptyl, octyl, nonyl, decyl, undecyl, dodecyl and their branched chain geometric isomers such as t-butyl and i-propyl. The term "cycloalkyl" should be understood as including cyclopropyl, cyclobutyl, cyclopentyl and cyclohexyl. The term "alkenyl" should be understood as including groups having the stated number of carbon atoms, their branched chain geometric isomers, and including one or more carbon to carbon double bonds such as ethenyl, propenyl, 1-butenyl, 2-butenyl, 2-methylpropenyl, 3,3-dimethyl-1-butenyl, 4-methyl-2-pentenyl, allenyl, 1,3-butadiene, 1,4-pentadiene, 1,3,5-hexatriene, isopropenyl and 1-isobutenyl. The term "heterocyclic" should be understood as including groups such as furyl, thienyl and pyridyl. The term "phenalkyl" should be understood as including groups such as benzyl, phenethyl, alpha-methyl-benzyl, 3-phenylpropyl and 2-phenylpropyl. The term "phenalkenyl" should be understood as including groups such as styrene, cinnamyl(1-phenyl-propene), 3-phenylpropene, 2-phenyl-2-butene, 4-phenyl-2-butene, 4-phenylbutene, 5-phenyl-1,3-pentadiene, 5-phenyl-1,4-pentadiene and 3-phenyl-1,2-propadiene. The term "phenoxy" should be understood as including groups such as 4-cyanophenoxy, 3-nitrophenoxy, 2,4-dichlorophenoxy and 3,5-dibromophenoxy. The term "benzyloxy" should be understood as including groups

such as 2-cyanobenzyloxy, 4-nitrobenzyloxy, 2,4-dibromobenzyloxy and 2,5-dichlorobenzyloxy.

Typical compounds within the scope of Formula I include, but are not limited to, the following:

O-trichloroacrylyl-1,1-dichloroacetonoxime;
O-acetyl-1,1-dichloroacetoneoxime;
O-crotonyl-1,1-dichloroacetoneoxime;
O-chloroacetyl-1,1-dichloroacetonoxime;
O-dichloroacetyl-1,1-dichloroacetoneoxime;
O-propionyl-1,1-dichloroacetoneoxime;
O-trichloroacetyl-1,1-dichloroacetoneoxime;
1,1-dichloroacetoneoxime-0-methylcarbonate;
1,1-dichloroacetoneoxime-0-ethylcarbonate;
1,1-dichloroacetoneoxime-0-isobutylcarbonate;
O-4-chlorobenzoyl-1,1-dichloroacetoneoxime;
O-benzoyl-1,1-dichloroacetoneoxime;
O-4-nitrobenzoyl-1,1-dichloroacetoneoxime;
O-2,4-dichlorobenzoyl-1,1-dichloroacetoneoxime;
O-3-chlorobenzoyl-1,1-dichloroacetoneoxime;
1,1-dichloroacetoneoxime-0-(N'-phenyl)carbamate;
O-2-furoyl-1,1-dichloroacetoneoxime;
O-4-methoxybenzoyl-1,1-dichloroacetoneoxime;
O-3,4-dichlorobenzoyl-1,1-dichloroacetoneoxime;
O-4-fluorobenzoyl-1,1-dichloroacetoneoxime;
O-4-methylbenzoyl-1,1-dichloroacetoneoxime;
1,1-dichloroacetoneoxime-0-(N'-methyl)carbamate;
O-butyryl-1,1-dichloroacetoneoxime;
O-valeryl-1,1-dichloroacetoneoxime;
O-(2-trichloroethoxycarbonyl)-1,1-dichloroacetoneoxime;
O-methylthiocarbonyl-1,1-dichloroacetoneoxime;
O-(2,4-hexadienoyl)-1,1-dichloroacetoneoxime;
O-(2-chlorobenzoyl)-1,1-dichloroacetoneoxime;
O-(2-methylbenzoyl)-1,1-dichloroacetoneoxime;

O-methacryloyl-1,1-dichloroacetoneoxime;
1,1,3,3-tetrachloroacetonoxime;
O-propionyl-1,1,3,3-tetrachloroacetoneoxime;
O-acetyl-1,1,3,3-tetrachloroacetoneoxime;
O-methoxycarbonyl-1,1,3,3-tetrachloroacetoneoxime;
O-benzoyl-1,1,3,3-tetrachloroacetoneoxime;
O-(4-methylbenzoyl)-1,1,3,3-tetrachloroacetoneoxime;
O-methacryloyl-1,1,3,3-tetrachloroacetoneoxime;
O-(N'-methylcarbamyl)-1,1,3,3-tetrachloroacetoneoxime; and
O-(3,5-dichlorobenzoyl)-1,1-dichloroacetoneoxime;
O-(3,3-dimethylacryloyl)-1,1-dichloroacetone-oxime;
O-(phenylacetyl)-1,1-dichloroacetoneoxime;
O-cyclohexyl-1,1-dichloroacetoneoxime;
O-(3-chloropropionyl)-1,1-dichloroacetoneoxime;
O-(2-chloropropionyl)-1,1-dichloroacetoneoxime;
O-(hydrocinnamoyl)-1,1-dichloroacetoneoxime;
O-(cinnamoyl)-1,1-dichloroacetoneoxime;
O-(methoxyacetyl)-1,1-dichloroacetoneoxime;
O-(benzoxycarbonyl)-1,1-dichloroacetoneoxime;
O-(3-trifluoromethylbenzoyl)-1,1-dichloroacetoneoxime;
O-(2,4-hexadienoyl)-1,1-dichloroacetoneoxime;
O-acryloyl-1,1-dichloroacetoneoxime;
O-(N'-cyclohexylcarbamyl)-1,1-dichloroacetoneoxime;
O-(4-chlorobenzoyl)-1,1,3-trichloroacetoneoxime;
O-propionyl-1,1,3,-trichloroacetoneoxime;
O-(methoxycarbonyl)-1,1,3-trichloroacetoneoxime;
O-(methacryloyl)-1,1,3-trichloroacetoneoxime;

O-(chloroacetyl)-1,1,3-trichloroacetoneoxime;
O-cyclohexylcarbonyl-1,1,3-trichloroacetoneoxime;
O-phenylacetyl-1,1,3-trichloroacetoneoxime;
O-(hydrocinnamoyl)-1,1,3-trichloroacetoneoxime;
O-(2-chloropropionyl)-1,1,3-trichloroacetoneoxime;
O-(3-chloropropionyl)-1,3,3-trichloroacetoneoxime;
O-benzoxycarbonyl-1,1,3-trichloroacetoneoxime;
O-(4-bromobenzoyl)-1,1,3-trichloroacetoneoxime;
O-(2-chlorobenzoyl)-1,1,3-trichloroacetoneoxime;
O-(3-chlorobenzoyl)-1,1,3-trichloroacetoneoxime;
O-(3,4-dichlorobenzoyl)-1,1,3-trichloroacetoneoxime;
O-(2,6-dichlorobenzoyl)-1,1,3-trichloroacetoneoxime;
O-(3,5-dichlorobenzoyl)-1,1,3-trichloroacetoneoxime;
O-benzoyl-1,1,3-trichloroacetoneoxime;
O-(4-methylbenzoyl)-1,1,3-trichloroacetoneoxime;
O-(4-nitrobenzoyl)-1,1,3-trichloroacetoneoxime;
O-acetyl-1,1,3-trichloroacetoneoxime;
O-n-butryl-1,1,3-trichloroacetoneoxime;
O-(lauroyl)-1,1,3-trichloroacetoneoxime;
O-(4-methoxybenzoyl)-1,1,3-trichloroacetoneoxime;
O-(2,4-hexadienoyl)-1,1,3-trichloroacetoneoxime;
O-(3,3-dimethylacryloyl)-1,1,3-trichloroacetoneoxime;
O-(ethoxycarbonyl)-1,1,3-trichloroacetoneoxime;
O-(N'-methylcarbamyl)-1,1,3-trichloroacetoneoxime;
O-acryloyl-1,1,3-trichloroacetoneoxime;

O-crotonyl-1,1,3-trichloroacetoneoxime;
O-(3-trifluoromethylbenzoyl)-1,1,3-trichloroacetoneoxime;
O-butyryl-1,1,3,3-tetrachloroacetoneoxime;
O-(2-chloropropionyl)-1,1,3,3-tetrachloroacetoneoxime;
O-(3-chloropropionyl)-1,1,3,3-tetrachloroacetoneoxime;
O-phenylacetyl-1,1,3,3-tetrachloroacetoneoxime;
O-(2,4-hexadienoyl)-1,1,3,3-tetrachloroacetoneoxime;
O-lauroyl-1,1,3,3-tetrachloroacetoneoxime;
O-(hydrocinnamoyl)-1,1,3,3-tetrachloroacetoneoxime;
O-methoxyacetyl-1,1,3,3-tetrachloroacetoneoxime;
O-benzoxycarbonyl-1,1,3,3-tetrachloroacetoneoxime;
O-ethoxycarbonyl-1,1,3,3-tetrachloroacetoneoxime;
O-cyclohexylcarbonyl-1,1,3,3-tetrachloroacetoneoxime;
O-chloroacetyl-1,1,3,3-tetrachloroacetoneoxime;
O-acryloyl-1,1,3,3-tetrachloroacetoneoxime;
O-crotonyl-1,1,3,3-tetrachloroacetoneoxime;
O-(3-trifluoromethylbenzoyl)-1,1,3,3-tetrachloroacetoneoxime; and
O-(N'-cyclohexylcarbamyl)-1,1,3,3-tetrachloroacetoneoxime.

Primarily because of their biocidal, fungicidal and/or pesticidal activity, preferred polyhaloketoximes are those of Formula I where one or more, preferably all, of the substituents accord to the following

definitions:-

X and X' are the same or different halogen selected from chloro or bromo;

R is methyl or halomethyl; and

$R^1$ is $(C_1-C_8)$alkyl optionally substituted with one or more of the same or different halogen;

$(C_3-C_6)$cycloalkyl,

straight or branched chain $(C_1-C_4)$alkoxy optionally substituted with one or more halogen;

an amino group of the formula $NHR^2$ where $R^2$ is hydrogen, straight or branched chain $(C_1-C_4)$alkyl, or aryl;

a thio group of the formula $SR^3$ where $R^3$ is straight or branched chain $(C_1-C_4)$alkyl;

$(C_2-C_6)$alkenyl, optionally substituted with one or more halogen;

aryl, optionally substituted with one or more of the same or different halogen, cyano, nitro, $(C_1-C_4)$alkyl, $(C_1-C_4)$alkoxy, or $(C_1-C_4)$haloalkyl;

a heterocyclic group;

phenalkyl where the alkyl moiety contains one to four carbon atoms; or

phenalkenyl where the alkenyl moiety contains two to five carbon atoms and the phenyl ring is optionally substituted with one or more of the same or different halogen, cyano, nitro, $(C_1-C_4)$alkyl, $(C_1-C_4)$alkoxy, or $(C_1-C_4)$haloalkyl.

More preferred are those compounds of Formula I where one or more, preferably all, of the substituents accord to the following definitions:

X and X' are chloro;
R is methyl, chloromethyl or dichloromethyl; and
$R^1$ is $(C_1-C_4)$alkyl optionally substituted with one to three of the same or different halogen;

$(C_3-C_6)$cycloalkyl;

$(C_1-C_4)$alkoxy;

an amino group of the formula $NHR^2$ where $R^2$ is $(C_1-C_4)$alkyl or phenyl;

a phenyl optionally substituted with one to three of the same or different halogen, nitro, $(C_1-C_4)$alkyl or $(C_1-C_4)$alkoxy;

furyl;

phenalkyl where the alkyl moiety contains one to four carbon atoms; or

phenalkenyl where the alkenyl moiety contains two to five carbon atoms.

All of the polyhaloketoximes of Formula I with the exception of the amino derivatives ($R^1$ is $NHR^2$) can be prepared by reacting an oxime of the following formula:

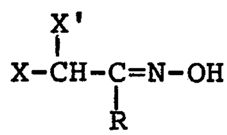

II

where X, X' and R are as defined above for Formula I with a compound of the formula:

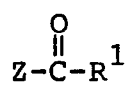

III

where $R^1$ is as defined above, except as previously stated, and Z is halo, preferably chloro, or $OCOR^1$ and $R^1$ is as defined above other than $NHR^2$. The reactions can be carried out in the presence of a solvent or solvent mixture and a suitable base.

Those polyhaloketoximes of Formula I where $R^1$ is $NHR^2$ can be prepared by reacting the oxime of Formula II with an isocyanate of the following formula

$$O{=}C{=}N{-}R^2 \qquad IV$$

where $R^2$ is as defined above for Formula I in the presence of a solvent or solvent mixture and optionally in the presence of a suitable catalyst.

In conducting the reaction between oximes of Formula II and compounds of Formula III, from about 1

to about 1.2 mole of the compounds of Formula III and from about 1 to about 1.2 mole of base are preferably employed per mole of oxime of Formula II. This reaction can be carried out at a temperature of from about 0°C to about 80°C. Generally, the reaction is exothermic so in most cases no heating is required. The polyhaloketoxime products of Formula I can be isolated by known methods. Suitable solvents for the reaction include inert organic solvents such as toluene, chloroform, methylene chloride and carbon tetrachloride. Preferred bases for the reaction are organic tertiary bases such as triethyl amine and pyridine, or inorganic bases such as sodium bicarbonate and potassium hydrocarbonate.

In preparing the amino derivatives of Formula I by reacting oximes of Formula II with isocyanates of Formula IV, from about 1 to about 4 moles of isocyanate are preferably employed per mole of oxime. This reaction can be carried out at 0°C to 110°C, preferably 0°C to 85°C. Preferred solvents are inert organic solvents such as acetone, acetonitrile, chloroform, carbon tetrachloride, tetrahydrofuran, dioxane, dimethylformamide and methylene chloride. Suitable catalysts, which are optionally employed, include triethylamine, pyridine and organotin compounds such as dibutyltin dilaurate. In isolating the amino derivatives of Formula I, the solvent is evaporated off and the residue worked up by known methods.

A typical example of an oxime of Formula II is 1,1-dichloroacetoneoxime which can be prepared by known procedures such as reacting 1,1-dichloroacetone with hydroxylamine in the presence of a base in a solvent or solvent mixture at from about 0°C to about 50°C. Other examples of the starting material of Formula II, such

as 1,1,3,3-tetrachloroacetoneoxime or 1,1,3-trichloroacetoneoxime can be prepared in a similar manner.

Examples of the compounds of Formula III that can be utilized in the above-described reaction include propionyl chloride, butyl chloride, dodecyl chloride, chloracetyl chloride, ethyl chloroformate, benzoyl chloride, 3,5-dichlorobenzoyl chloride, cinnamoyl chloride, methylthionyl chloride, 2-furoyl chloride, acetic anhydride and crotonyl anhydride. The compounds of Formula III are generally commercially available or can be prepared by known procedures.

Typical isocyanates of Formula IV which can be used in the above-described reaction include alkylisocyanates such as methyl isocyanate or ethyl isocyanate; and phenylisocyanate. The isocyanates of Formula IV are generally commercially available or can be prepared by known procedures.

The following examples are given for the purposes of illustration only. In Table I, embodiments of compounds of Formula I prepared by the above-described processes and constituting Examples 1 through 119 are set forth. Structures of the compounds of Examples 1 through 119 were confirmed by NMR analysis. Specific illustrative preparations of Examples 7, 10, 16, 23, 31, 34, 36, 37, 39 and 79 are set forth below.

TABLE I

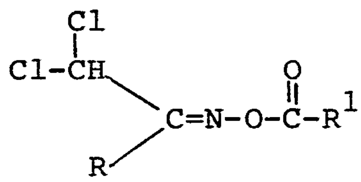

| Example No. | R | $R^1$ | B.P. or M.P.[1] |
|---|---|---|---|
| 1 | $CH_3$ | $-CCl{=}CCl_2$ | 80-110/0.1mm |
| 2 | $CH_3$ | $-CH_2(CH_2)_9CH_3$-<u>n</u> | oil |
| 3 | $CH_3$ | $-CH_3$ | oil |
| 4 | $CH_3$ | $-CH_2Cl$ | 38-40 |
| 5 | $CH_3$ | $-CH{=}CHCH_3$ | |
| 6 | $CH_3$ | $-CHCl_2$ | |
| 7 | $CH_3$ | $-CH_2CH_3$ | oil |
| 8 | $CH_3$ | $-CCl_3$ | oil |
| 9 | $CH_3$ | $-OCH_3$ | |
| 10 | $CH_3$ | $-OCH_2CH_3$ | oil |
| 11 | $CH_3$ | 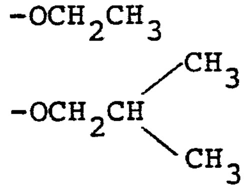 | oil |
| 12 | $CH_3$ | $-C_6H_4Cl$-4 | |
| 13 | $CH_3$ | $-C_6H_5$ | |
| 14 | $CH_3$ | $-C_6H_4NO_2$-4 | |
| 15 | $CH_3$ | $-C_6H_3Cl_2$-2,4 | oil |
| 16 | $CH_3$ | $-C_6H_4Cl$-3 | |
| 17 | $CH_3$ | $-NHC_6H_5$ | |
| 18 | $CH_3$ | 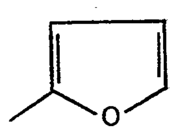 | |
| 19 | $CH_3$ | $-C_6H_4OCH_3$-4 | |
| 20 | $CH_3$ | $-C_6H_3Cl_2$-3,4 | |

| Example No. | R | $R^1$ | B.P. or M.P.[1] |
|---|---|---|---|
| 21 | $CH_3$ | $-C_6H_4F-4$ | |
| 22 | $CH_3$ | $-C_6H_4CH_3-4$ | |
| 23 | $CH_3$ | $-NHCH_3$ | |
| 24 | $CH_3$ | $-CH_2CH_2CH_3-\underline{n}$ | oil |
| 25 | $CH_3$ | $-CH_2CH_2CH_2CH_3-\underline{n}$ | oil |
| 26 | $CH_3$ | $-OCH_2CCl_3$ | |
| 27 | $CH_3$ | $-SCH_3$ | |
| 28 | $CH_3$ | $-CH=CH-CH=CHCH_3$ | |
| 29 | $CH_3$ | $-C_6H_4Cl-2$ | |
| 30 | $CH_3$ | $-C_6H_4CH_3-2$ | |
| 31 | $CH_3$ | $-C(CH_3)=CH_2$ | oil |
| 32 | $CH_3$ | $-C_6H_3Cl_2-3,5$ | 70-90 |
| 33 | $CH_3$ | $-CH=CH-C_6H_5$ | |
| 34 | $CHCl_2$ | $-CH_2CH_3$ | |
| 35 | $CHCl_2$ | $-CH_3$ | |
| 36 | $CHCl_2$ | $-OCH_3$ | |
| 37 | $CHCl_2$ | $-C_6H_5$ | |
| 38 | $CHCl_2$ | $-C_6H_4CH_3-4$ | |
| 39 | $CHCl_2$ | $-C(CH_3)=CH_2$ | |
| 40 | $CHCl_2$ | $-NHCH_3$ | |
| 41 | $CHCl_2$ | $-CH=CHC_6H_5$ | |
| 42 | $CHCl_2$ | $-C_6H_4Cl-4$ | 96-102 |
| 43 | $CHCl_2$ | $-C_6H_4Cl-3$ | 73-102 |
| 44 | $CHCl_2$ | $-C_6H_4Cl-2$ | 78-93 |
| 45 | $CHCl_2$ | $-C_6H_3Cl_2-2,4$ | 80-92 |
| 46 | $CHCl_2$ | $-C_6H_3Cl_2-3,4$ | 87-94 |
| 47 | $CHCl_2$ | $-C_6H_3Cl_2-3,5$ | 123-136 |
| 48 | $CHCl_2$ | $-C_6H_3Cl_2-2,6$ | 91-109 |
| 49 | $CHCl_2$ | $-C_6H_4OCH_3-3$ | 58-70 |
| 50 | $CHCl_2$ | $-C_6H_4OCH_3-4$ | 114-128 |
| 51 | $CHCl_2$ | $-C_6H_4CH_3-3$ | 84-97 |
| 52 | $CHCl_2$ | $-C_6H_4CH_3-2$ | 86-95 |

| Example No. | R | $R^1$ | B.P. or M.P.[1] |
|---|---|---|---|
| 53 | $CHCl_2$ | $-C_6H_4NO_2$-4 | 125-137 |
| 54 | $CHCl_2$ | $-C_6H_4NO_2$-3 | 122-127 |
| 55 | $CHCl_2$ | $-C_6H_4Br$-4 | 96-109 |
| 56 | $CHCl_2$ | 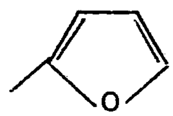 | 120-130 |
| 57 | $CHCl_2$ | $-C_6H_4F$-4 | |
| 58 | $CH_3$ | $-CH=C(CH_3)_2$ | |
| 59 | $CH_3$ | $-CH_2C_6H_5$ | |
| 60 | $CH_3$ | 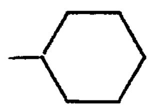 | |
| 61 | $CH_3$ | $-CH_2CH_2Cl$ | |
| 62 | $CH_3$ | $-C(H)ClCH_3$ | |
| 63 | $CH_3$ | $-CH_2CH_2C_6H_5$ | |
| 64 | $CH_3$ | $-CH=CHC_6H_5$ | 80-90 |
| 65 | $CH_3$ | $-CH_2OCH_3$ | oil |
| 66 | $CH_3$ | $-OCH_2C_6H_5$ | |
| 67 | $CH_3$ | $-C_6H_4CF_3$-3 | |
| 68 | $CH_3$ | $-CH=CHCH=CH-CH_3$ | |
| 69 | $CH_3$ | $-CH=CH_2$ | oil |
| 70 | $CH_3$ | -NH- 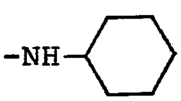 | |
| 71 | $CH_2Cl$ | $-C_6H_4Cl$-4 | 70-73 |
| 72 | $CH_2Cl$ | $-CH_2CH_3$ | oil |
| 73 | $CH_2Cl$ | $-OCH_3$ | oil |
| 74 | $CH_2Cl$ | $-C(CH_3)=CH_2$ | oil |
| 75 | $CH_2Cl$ | $-CH_2Cl$ | oil |
| 76 | $CH_2Cl$ | $-CH=CHC_6H_5$ | 94-98 |

| Example No. | R | $R^1$ | B.P. or M.P.[1] |
|---|---|---|---|
| 77 | $CH_2Cl$ | 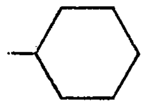 | 56-61 |
| 78 | $CH_2Cl$ | $-CH_2C_6H_5$ | oil |
| 79 | $CH_2Cl$ | $-CH_2CH_2C_6H_5$ | |
| 80 | $CH_2Cl$ | $-C(H)ClCH_3$ | oil |
| 81 | $CH_2Cl$ | $-CH_2CH_2Cl$ | oil |
| 82 | $CH_2Cl$ | $-OCH_2C_6H_5$ | oil |
| 83 | $CH_2Cl$ | $-C_6H_4F-4$ | oil |
| 84 | $CH_2Cl$ | $-C_6H_4Br-4$ | 64-70 |
| 85 | $CH_2Cl$ | $-C_6H_4Cl-2$ | oil |
| 86 | $CH_2Cl$ | $-C_6H_4Cl-3$ | 69-74 |
| 87 | $CH_2Cl$ | $-C_6H_3Cl_2-3,4$ | oil |
| 88 | $CH_2Cl$ | $-C_6H_3Cl_2-2,6$ | 96-101 |
| 89 | $CH_2Cl$ | $-C_6H_3Cl_2-3,5$ | 72-78 |
| 90 | $CH_2Cl$ | $-C_6H_5$ | oil |
| 91 | $CH_2Cl$ | $-C_6H_4CH_3-4$ | 76-82 |
| 92 | $CH_2Cl$ | $-C_6H_4NO_2-4$ | 98-104 |
| 93 | $CH_2Cl$ | $-CH_3$ | oil |
| 94 | $CH_2Cl$ | $-CH_2CH_2CH_3$ | oil |
| 95 | $CH_2Cl$ | $-CH_2(CH_2)_9-CH_3$ | oil |
| 96 | $CH_2Cl$ | $-C_6H_4OCH_3-4$ | oil |
| 97 | $CH_2Cl$ | $-CH=CHCH=CHCH_3$ | oil |
| 98 | $CH_2Cl$ | $-CH=C(CH_3)_2$ | oil |
| 99 | $CH_2Cl$ | $-OCH_2CH_3$ | oil |
| 100 | $CH_2Cl$ | $-NH-CH_3$ | oil |
| 101 | $CH_2Cl$ | $-CH=CH_2$ | oil |
| 102 | $CH_2Cl$ | $-CH=CHCH_3$ | oil |
| 103 | $CH_2Cl$ | $-C_6H_4CF_3-3$ | oil |
| 104 | $CHCl_2$ | $-CH_2CH_2CH_3$ | oil |
| 105 | $CHCl_2$ | $-C(H)ClCH_3$ | 50°C |
| 106 | $CHCl_2$ | $-CH_2CH_2Cl$ | oil |
| 107 | $CHCl_2$ | $-CH_2C_6H_5$ | |
| 108 | $CHCl_2$ | $-CH=CHCH=CHCH_3$ | |

| Example No. | R | $R^1$ | B.P. or M.P.[1] |
|---|---|---|---|
| 109 | $CHCl_2$ | $-CH_2(CH_2)_9-CH_3$-n | oil |
| 110 | $CHCl_2$ | $-CH_2CH_2C_6H_5$ | |
| 111 | $CHCl_2$ | $-CH_2OCH_3$ | |
| 112 | $CHCl_2$ | $-OCH_2C_6H_5$ | 86 |
| 113 | $CHCl_2$ | $-OCH_2CH_3$ | |
| 114 | $CHCl_2$ | 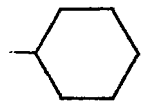 | 57-65 |
| 115 | $CHCl_2$ | $-CH_2Cl$ | |
| 116 | $CHCl_2$ | $-CH=CH_2$ | |
| 117 | $CHCl_2$ | $-CH=CHCH_3$ | |
| 118 | $CHCl_2$ | $-C_6H_4CF_3$-3 | 65 |
| 119 | $CHCl_2$ | -NH- 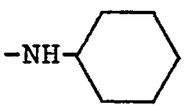 | |

[1] Boiling Point or Melting Point in °C.

## EXAMPLE 7

### Preparation of O-Propionyl-1,1-dichloroacetoneoxime

To a mixture of 1,1-dichloroacetoneoxime (5g, 35.2 mmole) and propionyl chloride (3.4g, 36 mmole) in toluene (90 ml) at 10-20°C with mechanical stirring, pyridine (3.68 ml, 35 mmole) was slowly added. The resulting white suspension was stirred at room temperature for 30 min. The reaction mixture was diluted with toluene (20 ml) and washed with $H_2O$ and brine. The organic layer was dried over $MgSO_4$ and filtered. The solvent was evaporated to afford 6.63g (95% based on oxime) of O-propionyl-1,1-dichloroacetoneoxime.

## EXAMPLE 10

### Preparation of O-Ethoxycarbonyl-1,1-dichloroacetoneoxime

A mixture of 1,1-dichloroacetoneoxime (7.10, 0.05 mmole) and ethyl chloroformate (6.54g, 0.06 mole) in toluene (100 ml) at 10 to 20°C was treated with triethylamine (9.1 ml, 0.06 mole) over 10 minutes with vigorous mechanical stirring. Stirring was continued for another 20 minutes. The reaction mixture was diluted with 25 ml of toluene, washed with water (2x20 ml) and brine. The organic layer was dried over $MgSO_4$ and filtered. The solvent was evaporated to afford 7.5g (75% of theory) of O-ethoxycarbonyl-1,1-dichloroacetoneoxime.

## EXAMPLE 16

### Preparation of O-3-Chlorobenzoyl-1,1-dichloroacetoneoxime

A mixture of 1,1-dichloroacetoneoxime (2.5g, 17.6 mmole) and 3-chlorobenzoyl chloride (3.15g, 18

mmole) in toluene (50 ml) at room temperature with mechanical stirring was treated slowly with triethylamine (2.5 ml, 18 mmole). After the addition was completed, the reaction mixture was stirred for another one hour. The resultant suspension was diluted with 50 ml of toluene and subsequently washed with $H_2O$, diluted $NaHCO_3$, $H_2O$ and brine. The organic layer was dried over $MgSO_4$ and filtered. The solvent was evaporated to afford 4.47g (90.5% of theory) of O-3-chlorobenzoyl-1,1-dichloroacetoneoxime.

EXAMPLE 23

Preparation of O-(N'-methylcarbamyl)-1,1-dichloroacetoneoxime

To a solution of 1,1-dichloroacetoneoxime (4g, 28.2 mmole) in methylene chloride (50 ml) was added at room temperature (RT) methylisocyanate (3.42g, 60 mmole) and 10 drops of triethylamine. The reaction mixture was stirred at RT for 48 hours. The volatiles were evaporated on a rotary evaporator to afford 5.0g (96% of theory) of O-(N'-ethylcarbamyl)-1,1-dichloroacetoneoxime.

EXAMPLE 31

Preparation of O-Methyacryloyl-1,1-dichloroacetoneoxime

To a mixture of 1,1-dichloroacetoneoxime (4g, 28.2 mmole) and methacryloyl chloride (4.35g, 30 mmole) in toluene (50 ml) was slowly added pyridine (3 ml, 35 mmole) with mechanical stirring at 5 - 10°C. After addition, the reaction mixture was stirred at room temperature for one hour. The resultant white suspension was diluted with EtOAc and washed subsequently with $H_2O$, dil $NaHCO_3$ and $H_2O$. After brine, the organic layer was dried over $Na_2SO_4$.

Evaporation of solvents afforded 4.1g (70% of theory) of O-methacryloyl-1,1-dichloroacetoneoxime.

EXAMPLE 34

Preparation of O-Propionyl-1,1,3,3-Tetrachloroacetoneoxime

A similar procedure to that stated in Example 7 was followed. 4g (18.96 mmole) of 1,1,3,3-tetrachloroacetoneoxime and 1.81g (19.5 mmole) of propionyl chloride in toluene (75 ml) were treated with pyridine (1.6 ml, 20 mmole). After work-up procedure, 4.26g (84% of theory) of O-propionyl-1,1,3,3-tetrachloroacetoneoxime was obtained.

EXAMPLE 36

Preparation of O-Methoxycarbonyl-1,1,3,3-tetrachloroacetoneoxime

A similar procedure to that stated in Example 10 was followed. Starting with 4g (18.96 mmole) of 1,1,3,3-tetrachloroacetoneoxime, 1.85g (19.5 mmole) of methyl chloroformate and 1.6 ml (20 mmole) of pyridine in toluene (60 ml), the product, 4.22g (83% of theory) of carbonate O-methoxycarbonyl-1,1,3,3-tetrachloroacetoneoxime was obtained.

EXAMPLE 37

Preparation of O-Benzoyl-1,1,3,3-Tetrachloroacetoneoxime

A similar procedure to that stated in Example 16 was followed. Starting with 4g (18.96 mmole) of 1,1,3,3-tetrachloroacetoneoxime, 2.74g (19.5 mmole) of benzoyl chloride and 1.6 ml (20 mmole) of pyridine, the product, 5.22g (87.4% of theory) O-benzoyl-1,1,3,3-tetrachloroacetoneoxime was obtained.

## EXAMPLE 39

### Preparation of O-Methacryloyl-1,1,3,3-Tetrachloroacetoneoxime

A similar procedure to that stated in Example 31 was followed. Starting with 3g (14.2 mmole) of 1,1,3,3,-tetrachloroacetoneoxime, 2.18g (15 mmole) of methacryloyl chloride and 1.3 ml (16 mmole) of pyridine in toluene (30 ml), 3.85g (97% of theory) of O-methacryloyl-1,1,3,3-tetrachloroacetoneoxime was obtained.

## EXAMPLE 79

### Preparation of O-Hydrocinnamoyl-1,1,3-trichloroacetoneoxime

To a mixture of 1,1,3-trichloroacetoneoxime (4g, 23 mmole) and hydrocinnamoyl chloride (4.27g, 25 mmole) in methylene chloride (100 ml) at 0-5°C with magnetic stirring, pyridine (2g, 25 mmole) was dropwise added.

After addition, the reaction mixture was stirred at 0-5°C for 10 minutes and at room temperature for 60 minutes.

The reaction mixture was transferred to a separatory funnel and washed with $H_2O$ and brine. The organic layer was dried over $MgSO_4$ and filtered. The solvent was evaporated to afford 7.0g (99% based on oxime) of O-hydrocinnamoyl-1,1,3-trichloroacetoneoxime.

The novel polyhaloketoximes of this invention are biocidally active compounds and as such are suitable for the control of living organisms and particularly microorganisms.

Primarily because of their activity, polyhaloketoximes most preferred as biocides are those compounds of Formula I where one or more, preferably all, of the substituents accord to the following definitions:

X and X' are chloro;
R is methyl, chloromethyl or dichloromethyl; and
$R^1$ is $(C_1-C_4)$-alkyl optionally substituted with one to three of the same or different halogen,

cyclohexyl,

$(C_1-C_4)$-alkoxy,

$(C_2-C_5)$-alkenyl,

phenyl, optionally substituted with one to three of the same or different halogen, $(C_1-C_4)$-alkyl or $(C_1-C_4)$-alkoxy,

furyl,

phenalkyl where the alkyl moiety contains one to four carbon atoms, or

phenalkenyl where the alkenyl moiety contains two to five carbon atoms.

Antibacterial and fungicidal activity was evaluated by the Serial Dilution Test (Broth Titer Test) wherein a series of broths containing varying dilutions of a test compound and an organism are halved starting with 500 ppm. The values obtained, which are shown in Table II, rounded off to the nearest whole number, represent the minimum inhibitory concentration in parts per million at which the compound under evaluation renders complete control of the organism.

The following fungi and bacteria were employed in this test:

| Fungi | Bacteria |
|---|---|
| *Aspergillis niger* (ANIG) | *Escherichia coli* (ECOL) |
| *Aureobasidium pullulans* (APUL) | *Pseudomonas aeruginosa* (PSAE) |
| *Candida albicans* (CALB) | *Pseudomonas fluorescens* (PSFL) |
| | *Staphylococcus aureus* (SAUR) |

TABLE II

Microbiological Activity

| Example No. | ANIG | APUL | CALB | ECOL | PSAE | PSFL | SAUR |
|---|---|---|---|---|---|---|---|
| 1 | 125 | 63 | 125 | >[1] | 500 | > | 500 |
| 2 | > | -[2] | - | - | - | > | - |
| 3 | <4 | <4 | 8 | > | 250 | 125 | 500 |
| 4 | > | - | - | - | - | > | - |
| 5 | 8 | 8 | 63 | > | > | > | > |
| 6 | 63 | 63 | 63 | > | > | > | > |
| 7 | <4 | <4 | <4 | > | 500 | 125 | 500 |
| 8 | > | - | - | - | - | > | - |
| 9 | <4 | <4 | 31 | > | 500 | 500 | 500 |
| 10 | <4 | <4 | <4 | > | > | > | 500 |
| 11 | 31 | 63 | 63 | > | > | > | > |
| 12 | <4 | 63 | 31 | > | > | > | 250 |
| 13 | <4 | 8 | 31 | > | > | > | 500 |
| 14 | 31 | 16 | 31 | > | > | ≥ | 500 |
| 15 | 8 | 16 | 250 | > | > | > | > |
| 16 | 8 | 31 | 63 | > | > | 500 | 500 |
| 17 | > | - | - | - | - | > | - |
| 18 | <4 | 8 | 16 | > | > | 500 | 500 |
| 19 | 16 | 16 | 250 | > | > | 500 | > |
| 20 | <4 | 8 | 16 | > | > | 500 | > |
| 21 | <4 | <4 | 31 | > | > | 500 | 500 |
| 22 | <4 | <4 | 250 | > | > | 500 | > |
| 23 | 500 | - | - | - | - | > | - |
| 24 | <4 | 8 | <4 | > | > | 500 | > |

| Example No. | ANIG | APUL | CALB | ECOL | PSAE | PSFL | SAUR |
|---|---|---|---|---|---|---|---|
| 25 | 16 | 16 | <4 | > | > | 500 | > |
| 26 | 125 | 63 | 31 | > | > | > | > |
| 27 | 31 | 250 | 125 | > | > | > | > |
| 28 | 8 | 8 | 63 | > | > | > | > |
| 29 | 8 | 16 | 250 | > | > | > | 500 |
| 30 | 16 | 250 | > | > | > | > | 500 |
| 31 | <4 | 8 | 31 | > | > | > | 500 |
| 32 | 250 | - | - | - | - | > | - |
| 33 | 8 | 16 | > | > | > | > | > |
| 34 | 63 | <4 | <4 | > | > | > | > |
| 35 | 63 | 8 | 8 | > | > | > | > |
| 36 | 125 | 16 | 16 | > | > | 500 | > |
| 37 | <4 | <4 | 8 | > | > | > | <4 |
| 38 | <4 | <4 | 8 | > | > | > | <4 |
| 39 | > | - | - | - | - | 500 | - |
| 40 | > | - | - | - | - | 500 | - |
| 41 | <4 | 8 | 125 | > | > | > | 31 |
| 42 | <4 | <4 | > | > | > | > | 8 |
| 43 | <4 | <4 | > | > | > | > | 8 |
| 44 | 8 | <4 | > | > | > | > | 8 |
| 45 | 16 | <4 | > | > | > | > | 31 |
| 46 | 16 | <4 | > | > | > | > | 63 |
| 47 | 250 | - | - | - | - | > | - |
| 48 | > | - | - | - | - | > | - |
| 49 | <4 | <4 | > | > | > | > | 31 |
| 50 | <4 | - | - | - | - | > | - |
| 51 | <4 | 8 | > | > | > | > | 8 |
| 52 | 16 | 8 | > | > | > | > | <4 |
| 53 | 500 | - | - | - | - | > | - |

| Example No. | ANIG | APUL | CALB | ECOL | PSAE | PSFL | SAUR |
|---|---|---|---|---|---|---|---|
| 54 | 500 | - | - | - | - | > | - |
| 55 | 125 | - | - | - | - | > | - |
| 56 | 8 | 16 | > | > | > | > | 63 |
| 57 | <4 | <4 | 500 | > | > | 500 | <4 |
| 58 | <4 | 250 | > | > | > | > | > |
| 59 | 16 | 16 | > | > | > | > | > |
| 60 | 8 | 16 | > | > | > | > | 500 |
| 61 | <4 | <4 | 250 | 500 | 500 | 500 | 500 |
| 62 | <4 | 125 | 500 | > | > | 500 | > |
| 63 | 8 | 16 | > | > | > | 500 | 500 |
| 64 | <4 | 16 | > | > | > | > | > |
| 65 | 125 | - | - | - | - | 500 | - |
| 66 | 8 | 63 | > | > | > | > | > |
| 67 | 63 | 31 | > | > | > | > | > |
| 68 | 8 | 16 | > | > | > | > | > |
| 69 | 31 | 16 | > | > | > | > | > |
| 70 | > | - | - | - | - | > | - |
| 71 | <4 | <4 | 63 | > | > | > | 31 |
| 72 | <4 | <4 | 16 | 125 | 125 | 125 | 63 |
| 73 | <4 | - | - | - | - | 500 | - |
| 74 | <4 | <4 | 125 | > | 250 | 250 | 63 |
| 75 | 250 | - | - | - | - | 500 | - |
| 76 | 8 | <4 | > | > | > | 500 | 31 |
| 77 | <4 | <4 | > | > | > | > | 63 |
| 78 | <4 | 31 | 250 | > | 500 | 500 | 500 |
| 79 | <4 | <4 | 125 | > | 500 | 500 | 63 |
| 80 | > | - | - | - | - | 500 | - |
| 81 | <4 | <4 | 63 | 250 | 250 | 250 | 125 |
| 82 | 8 | <4 | 250 | > | 500 | 500 | 500 |

| Example No. | ANIG | APUL | CALB | ECOL | PSAE | PSFL | SAUR |
|---|---|---|---|---|---|---|---|
| 83 | <4 | <4 | > | > | 500 | 500 | 16 |
| 84 | <4 | <4 | > | > | > | 500 | 63 |
| 85 | <4 | <4 | 125 | > | 250 | 500 | 63 |
| 86 | <4 | <4 | > | > | > | 500 | 63 |
| 87 | - | - | - | - | - | - | - |
| 88 | > | - | - | - | - | > | - |
| 89 | <4 | <4 | > | > | > | > | 31 |
| 90 | <4 | <4 | 125 | > | 250 | 250 | 31 |
| 91 | <4 | <4 | > | > | > | > | > |
| 92 | 16 | - | - | - | - | > | - |
| 93 | <4 | <4 | 31 | 250 | 250 | 125 | 500 |
| 94 | <4 | <4 | 125 | 500 | 500 | 125 | 500 |
| 95 | 63 | 250 | > | > | > | 250 | > |
| 96 | <4 | <4 | > | > | > | > | > |
| 97 | <4 | <4 | > | > | > | 250 | > |
| 98 | <4 | <4 | 250 | > | > | 500 | > |
| 99 | <4 | <4 | 250 | > | > | > | > |
| 100 | 125 | - | - | - | - | > | - |
| 101 | 31 | - | - | - | - | > | - |
| 102 | <4 | <4 | 250 | > | > | > | > |
| 103 | <4 | <4 | > | > | > | > | > |
| 104 | 63 | 16 | 250 | > | > | 500 | > |
| 105 | > | - | - | - | - | 500 | - |
| 106 | 500 | - | - | - | - | 500 | - |
| 107 | 250 | - | - | - | - | 500 | - |
| 108 | 8 | 16 | 500 | > | > | 500 | > |
| 109 | > | - | - | - | - | > | - |
| 110 | 31 | <4 | > | > | > | > | > |
| 111 | 500 | 250 | 250 | > | > | > | > |

| Example No. | ANIG | APUL | CALB | ECOL | PSAE | PSFL | SAUR |
|---|---|---|---|---|---|---|---|
| 112 | 125 | - | - | - | - | > | - |
| 113 | 125 | - | - | - | - | 500 | - |
| 114 | 16 | 16 | > | > | > | > | > |
| 115 | > | - | - | - | - | 500 | - |
| 116 | 31 | 31 | 250 | > | > | 500 | > |
| 117 | 8 | 16 | > | > | 500 | 500 | 500 |
| 118 | 63 | 8 | > | > | > | > | > |
| 119 | 250 | - | - | - | - | > | - |

[1] Greater than 500 ppm

[2] No data recorded.

The polyhaloketoximes of this invention also exhibit activity against assorted phytopathogenic fungi. Primarily because of their activity, polyhaloketoximes most preferred as fungicides are those compounds of Formula I where

X and X' are chloro;

R is methyl or dichloromethyl; and

$R^1$ is an amino group of the formula $NHR^2$ where $R^2$ is

phenyl;

phenyl, optionally substituted with from one to three of the same or different halogen, nitro, $(C_1-C_4)$alkyl or $(C_1-C_4)$-alkoxy; or

phenalkenyl where the alkenyl moiety contains two to five carbon atoms.

In evaluating the compounds of this invention, potted test plants in proper condition of growth for susceptibility to the fungal disease are sprayed to run-off with suspensions of the compound to be evaluated in a dosage series on a moving belt at a carrier volume of about 150 gallons/acre.

The sprayed plants are allowed to dry. The proper plants are then inoculated with the fungal spores which are allowed to incubate until the disease has developed. Counts of lesions are made.

The following specific test methods were employed in evaluating the fungicidal activity of the compounds of this invention.

## EXAMPLE 120

Cucumber Anthracnose (Colletotrichum Lagenarium)

Cucumber (var. 'Marketer') seedlings are 14 days old when treated.

Colletotrichum lagenarium is cultured on green

bean agar (GBA) in petri plates for 7 days under dark conditions. Petri plates of GBA are inoculated by spreading inoculum over medium with an inoculating needle. Spores are harvested from plates by adding deionized water to the GBA plates. The agar surface is scraped with a rubber policeman or similar blunt object. The spore suspension is filtered through cheesecloth to remove mycelial and agar fragments and then adjusted to a concentration of $1\text{-}2x10^5$ spores per ml.

Treated cucumber plants are inoculated by spraying the leaves (especially the underside) until a uniform film of inoculation is observed on the plant. Inoculated plants are incubated in a humid environment at 70°-75°F. for 72 hours. They are removed from the humid environment, allowed to dry, and placed under existing greenhouse conditions.

Treatment comparisons are made 10-14 days after inoculation. Typical anthracnose symptoms are small spots on the foliage that begin as small yellowish or water-soaked areas that enlarge rapidly and become necrotic.

EXAMPLE 121

Cucumber Downey Mildew (*Pseudoperonspora cubensis*)

Cucumber (var. "Marketer") seedlings are grown for three weeks at 65-75°F in moderate light before use.

*Pseudoperonospora cubensis* is cultured on cucumber seedlings for 7 days at 65-75°F in moderate light (alternating light and dark periods). Spores are harvested by adding deionized water and shaking leaves in a quart jar. The spore suspension is filtered

through cheesecloth to remove plant debris and adjusted to a concentration of 1 x $10^5$ spores per ml.

The cucumber plants are inoculated by spraying the under side of the leaves with a DeVilbiss atomizer until small droplets are observed on the leaves. The inoculated leaves are incubated in a mist chamber for 24 hours at 70°F and then subsequently incubated for 6-7 days in a controlled temperature room under mist at 65-75°F.

Treatment comparisons are made 7 days after inoculation by estimating percent infected leaf surface. Symptoms appear as yellowing of the upper leaf surface and grey sporulating areas on the lower leaf surface.

## EXAMPLE 122

### Rice Blast (*Piricularia oryzae*)

Rice plants (var. Calrose) are trimmed to a height of approximately 5 inches, 24 hours prior to chemical application. This procedure provides plants of uniform height and permits rapid inoculation of treated plants. Rice plants are inoculated by spraying the leaves and stems with an air brush until a uniform film of inoculum is observed on the leaves. The inoculated plants are incubated in a humid environment (75°-85°F) for 24 hours prior to being placed in a greenhouse environment. Treatment comparisons are made 7 to 8 days after inoculation. Initial rice blast lesions appear as small brown necrotic spots on the foliage. The typical lesion is eliptical, 1 to 2 cm. long with a large necrotic gray center and brown margins.

EXAMPLE 123

Rice Sheath Blight

(*Pellicularia filamentosa* f. sp. *sasakii*

Ten-day-old rice seedlings (var. "Lebonnet") are trimmed to a uniform height 24 hours prior to chemical application.

*Pellicularia filamentosa* f. sp. *sasakii* is cultured on an autoclaved mixture of crushed rice seeds/potato dextrose broth (100 g/30 ml) in a 500 ml wide-mouth Erlenmeyer flask. Ten-day-old cultures are run through a blender to produce uniform inoculum. Approximately one teaspoon of inoculum is spread among the rice seedlings on the soil surface of each pot (3" diameter). Inoculated seedlings are incubated for five days in a humidity cabinet (85-90°F). Treatment comparisons are made immediately after removing the seedlings from the cabinet. The disease appears as a white furry or web-like mycelial growth extending from the soil surface up the stems of the seedlings.

EXAMPLE 124

Tomato Late Blight

(*Phytophthora infestans*)

Tomato (var. Rutgers) seedlings, 2.5 to 3 inches tall, are fertilized with a water soluble fertilizer 4 to 5 days prior to chemical application to promote rapid succulent growth and better symptom expression. The spore suspension is applied with a DeVilbiss atomizer at 8 to 10 psi air pressure onto the leaf undersurface until fine droplets are formed. Inoculated seedlings are placed in a humid environment at 60°-65°F. for 40 to 45 hours, prior to being placed in the greenhouse at 70°-75°F. Treatment comparisons are made 5 to 6 days after inoculation. Initially,

typical tomato late blight symptoms appear as irregular, greenish-black, water-soaked patches which enlarge and become brown, with a firm corrugated surface. Severe infection will resemble frost damage.

EXAMPLE 125

Wheat Powdery Mildew (*Erysiphe graminis*)

"Pennoll" wheat seedlings (7-14 days old) are trimmed 24 hours prior to chemical application to provide a uniform plant height and to facilitate uniform inoculation.

Wheat powdery mildew is cultured on wheat seedlings in a controlled temperature room at 65-75°F.

Mildew spores are shaken from culture plants onto seedlings. Inoculated seedlings are kept in the controlled temperature room and sub-irrigated. Disease control is rated 8-10 days after inoculation.

EXAMPLE 126

Wheat Stem Rust

(*Puccinia graminis F. sp. tritici race 15 B-2*)

Seven-day-old wheat plants (var. Wanzer) are trimmed to approximately 2.5 inches, 24 hours prior to chemical application to provide a uniform plant height and to facilitate uniform inoculation. Wheat stem rust is cultured on wheat seedlings (var. Wanzer) for a period of 14 days under existing greenhouse conditions. Wheat plants are inoculated by applying the stem rust spore suspension, until run-off, with a DeVilbiss atomizer at 5 psi air pressure. After inoculation, the plants are placed into a humid environment at approximately 68°F. A timer is used to permit 12 hours of continuous darkness followed by a minimum of 3 to 4 hours of light with an intensity of 500 foot candles.

The temperature in the chamber should not exceed 85°F. At the end of the light period, the plants are placed into a greenhouse environment. The plants are permitted to grow under greenhouse conditions for a period of 2 weeks prior to making treatment comparisons. Wheat stem rust is characterized by brick red spores in irregularly shaped sori on the leaves and stems of the wheat seedlings.

TABLE III

| Example No. | Phytopathogenic Fungi Activity[1] | | | | | | |
|---|---|---|---|---|---|---|---|
| | CA[2] | CDM | RB | RSB | TLB | WPM | WSR |
| 1 | -[3] | 0 | 0 | 0 | 0 | 75 | 50 |
| 2 | - | 0 | 0 | 0 | 0 | 50 | 0 |
| 3 | 0 | 20 | 20 | 0 | 0 | 50 | 0 |
| 4 | - | 0 | 0 | 0 | 0 | 50 | 0 |
| 5 | 0 | 0 | 20 | 0 | 95 | 0 | 0 |
| 6 | - | 0 | 50 | 0 | 0 | 75 | 50 |
| 7 | 0 | 0 | 0 | 0 | 100 | 0 | 0 |
| 8 | 0 | 0 | 0 | 0 | 0 | 0 | 0 |
| 9 | 0 | 0 | 0 | 0 | 100 | 0 | 0 |
| 10 | 0 | 0 | 0 | 0 | 100 | 0 | 0 |
| 11 | 0 | 0 | 40 | 0 | 95 | 80 | 0 |
| 12 | 0 | 0 | 85 | 0 | 90 | 0 | 0 |
| 13 | 0 | 0 | 75 | 0 | 100 | 0 | 90 |
| 14 | 90 | 0 | 85 | 0 | 100 | 80 | 50 |
| 15 | 0 | 50 | 0 | 0 | 100 | 0 | 90 |
| 16 | - | 100 | 0 | 0 | 0 | 50 | 100 |
| 17 | 0 | 80 | 0 | 0 | 100 | 50 | 95 |
| 18 | 0 | 0 | 50 | 0 | 100 | 0 | 70 |
| 19 | 0 | 0 | 0 | 0 | 100 | 80 | 80 |
| 20 | 60 | 0 | 0 | 0 | 100 | 50 | 90 |
| 21 | 0 | 0 | 0 | 0 | 95 | 50 | 0 |
| 22 | 0 | 20 | 0 | 0 | 0 | 0 | 60 |
| 23 | 0 | 0 | 0 | 0 | 0 | 0 | 0 |
| 24 | 0 | 0 | 50 | 0 | 0 | 0 | 0 |
| 25 | 0 | 0 | 95 | 0 | 0 | 0 | 0 |
| 26 | - | 0 | 0 | 0 | 0 | 0 | 50 |
| 27 | - | 0 | 0 | 0 | 0 | 0 | 50 |
| 28 | - | 0 | 0 | 0 | 0 | 0 | 75 |
| 29 | - | 0 | 0 | 0 | 0 | 0 | 50 |
| 30 | - | 0 | 0 | 0 | 0 | 0 | 50 |
| 31 | - | 90 | 0 | 0 | 0 | 50 | 75 |

| Example No. | Phytopathogenic Fungi Activity[1] | | | | | | |
|---|---|---|---|---|---|---|---|
| | CA[2] | CDM | RB | RSB | TLB | WPM | WSR |
| 32 | - | 0 | 0 | 0 | 0 | 90 | 50 |
| 33 | - | 0 | 0 | 0 | 0 | - | 50 |
| 34 | 0 | 0 | 0 | 0 | 0 | 0 | 0 |
| 35 | 0 | 40 | 0 | 0 | 0 | 0 | 0 |
| 36 | 0 | 0 | 0 | 0 | 0 | 0 | 0 |
| 37 | 80 | 0 | 80 | 0 | 0 | 0 | 100 |
| 38 | 0 | 98 | 90 | 0 | 0 | 80 | 100 |
| 39 | - | 0 | 0 | 0 | 0 | 50 | 75 |
| 40 | - | 0 | 0 | 0 | 0 | 75 | 50 |
| 41 | - | 0 | 0 | 0 | 0 | 98 | 95 |
| 42 | - | 0 | 0 | 0 | 0 | 100 | 75 |
| 43 | - | 0 | 0 | 50 | 0 | 95 | 50 |
| 44 | - | 0 | 0 | 0 | 0 | 100 | 75 |
| 45 | - | 0 | 0 | 0 | 50 | 99 | 75 |
| 46 | - | 0 | 0 | 0 | 0 | 98 | 75 |
| 47 | - | 0 | 50 | 0 | 0 | 99 | 75 |
| 48 | - | 0 | 0 | 0 | 0 | 95 | 75 |
| 49 | - | 0 | 50 | 0 | 0 | 50 | 50 |
| 50 | - | 0 | 50 | 50 | 95 | 75 | 50 |
| 51 | - | 99 | 0 | 0 | 0 | 90 | 50 |
| 52 | - | 0 | 0 | 0 | 0 | 75 | 75 |
| 53 | - | 70 | 0 | 0 | 0 | 50 | 50 |
| 54 | - | 0 | 0 | 0 | 95 | 75 | 50 |
| 55 | - | 0 | 0 | 0 | 0 | 90 | 75 |
| 56 | - | 50 | 0 | 0 | 0 | 75 | 50 |
| 57 | - | 0 | 0 | 0 | 0 | 0 | 50 |
| 66 | - | - | - | - | - | 0 | 0 |
| 67 | - | - | - | - | - | 0 | 0 |
| 68 | - | - | - | - | - | 0 | 0 |
| 69 | - | - | - | - | - | 0 | 0 |
| 70 | - | 0 | 0 | 80 | 0 | 0 | 75 |

| Example No. | Phytopathogenic Fungi Activity[1] | | | | | | |
|---|---|---|---|---|---|---|---|
| | CA[2] | CDM | RB | RSB | TLB | WPM | WSR |
| 99 | - | - | - | - | - | 0 | 0 |
| 101 | - | - | - | - | - | 0 | 75 |
| 112 | - | - | - | - | - | 50 | 0 |
| 113 | - | - | - | - | - | 50 | 0 |
| 114 | - | - | - | - | - | 0 | 0 |
| 115 | - | - | - | - | - | 0 | 0 |
| 119 | - | 0 | 0 | 50 | 0 | 50 | 50 |

[1] Percent control at 300 ppm.

[2] CA = Cucumber Anthracnose
CDM = Cucumber Downy Mildew
RB = Rice Blast
RSB = Rice Sheath Blight
TLB = Tomato Late Blight
WPM = Wheat Powdery Mildew
WSR = Wheat Stem Rust

[3] No data recorded

The polyhaloketoximes of the invention are also active as pesticides. Primarily because of their activity, polyhaloketoximes most preferred as insecticides are those compounds of Formula I where:

X and X' are chloro;

R is methyl, chloromethyl or dichloromethyl; and $R^1$ is an amino group having the formula $NHR^2$ where $R^2$ is $(C_1-C_4)$alkyl,

cyclohexyl, or

phenyl optionally substituted with from one to three of the same or different halogen or $(C_1-C_4)$alkyl.

Certain compounds were effective against arthropods (in varying stages of development), members of the Class Arachnoidea which includes the Order Acarina, as represented by mites and ticks, and the Class Insecta, the insects. Further, certain compounds of this invention were shown to possess nematocidal activity.

Initial evaluations were made on the following pests:

| Code Symbol | Common Name | Latin Name |
|---|---|---|
| SAW | Southern armyworm | *Spodoptera eridania* |
| MBB | Mexican bean beetle | *Epilachna varivestis* |
| BW | Boll weevil | *Anthromonus grandis grandis* |
| CRW | Southern corn root-worm | *Diabrotica undecimpunctata howardi* |

| | | |
|---|---|---|
| GPA | Green peach aphid | Myzus persicae |
| MTA | Two-spotted spider mite | Tetranychus Urticae |
| NEM | Southern root-knot nematode | Meloidogyne incognita |

A test solution containing 600 ppm of test compound is made by dissolving the test compound in a solvent (acetone:methanol, 1:1), adding a surfactant and then water to give an acetone:methanol:water system of 10:10:80. A 1:1 mixture of an alkylarylpolyether-alcohol (commercially available under the trademark Triton® X-155) and a modified phthalic glycerol alkyl resin (commercially available under the trademark Triton® B-1956) can be utilized at the equivalent of one ounce per 100 gallons of test solution as a surfactant.

For the mite test, infested bean (Phaseolus limeanus) leaf discs (1.25 inches in diameter) containing about 50 mites and for green peach aphid tests, infested brocoli (Brassica oleracea italica) leaves or portions thereof containing about 50 aphids are placed in a Petri dish lid on a moistened piece of cotton. The leaves are then sprayed with the test solution using a rotating turntable. They are held for 24 hours and then the percent kill is determined.

For the bean beetle and armyworm test, detached bean leaves on pieces of moistened filter paper are sprayed as above for the mite test in similar dishes and allowed to dry. One such dish is infested with 10 third instar Mexican bean beetle larvae, which another is infested with 10 third instar southern armyworm larvae. The dishes are covered. After holding for 48 hours, the percent kill is obtained.

For the nematode test, soil is homogeneously inoculated with a macerated blend of tomato roots heavily knotted with the root knot nematode. Ten milliliters of the test solution are added to 200 milliliters of the inoculated soil in a 16 oz. jar to give a concentration by volume of about 30 ppm. The jar is then shaken to insure thorough mixing, immediately uncapped, and allowed to air for 24 hours. The soil is then placed into a 3 inch plastic pot after which time 3 cucumber (Cucumis sativus) seeds are planted. About 23 days thereafter, the cucumber plants are removed from the soil and the root system examined for the presence of knots. A total of 25 knots or less is considered as a measure of control.

Ovicidal and larvicidal tests are conducted on representative compounds of this invention. These compounds demonstrate ovicidal and larvicidal activity.

For tests involving the southern corn rootworm (Diabrotica undecimpunctata howardi) ova and larvae, two layers of 4.25 cm. filter papers are placed in small, Petri dishes, and sprayed on the turntable with a 600 ppm solution of the test compound and air dried. About 100 eggs in about one milliliter of water are pipetted onto the filter paper and the dishes covered. These are held for 6 days and examined under the microscope. The percent kill values for ova and larvae are determined.

For the boll weevil test, cotton (Gossypium hirsutum var. Acala) seedlings in 3-inch pots are sprayed to runoff with the test solutions using a DeVilbiss atomizer at 20 psi. When dry, each plant was placed in a plastic box (7.5" long x 5.25" wide x 3.75" deep). Ten young adult Boll Weevils are placed in each plastic box containing the plant that has been allowed

to dry. Each box was then sealed with a lid equipped with screened ventilation holes. Plants are watered as needed and maintained under continuous fluorescent lights at 80±5°F on open shelves. Six days after treatment the percent mortality is determined.

Table IV gives the results of the foregoing pesticidal evaluations.

TABLE IV

| Example No. | Pesticidal Results, % Control | | | | | | |
|---|---|---|---|---|---|---|---|
| | SAW | MBB | BW | CRW[1] | GPA | MTA | NEM[1] |
| 1 | -[2] | - | - | - | - | - | - |
| 2 | 0 | 0 | 0 | 0 | 0 | 0 | 0 |
| 3 | 0 | 30 | 20 | - | 0 | 0 | - |
| 4 | 0 | 10 | 0 | - | 0 | 0 | - |
| 5 | 0 | 0 | 40 | - | 0 | 0 | - |
| 6 | 0 | 0 | 0 | - | 0 | 0 | - |
| 7 | 0 | 0 | 0 | - | 0 | 100 | - |
| 8 | 0 | 0 | 20 | - | 0 | 0 | - |
| 9 | 0 | 0 | 0 | - | 0 | 76 | - |
| 10 | 0 | 0 | 0 | - | 0 | 0 | - |
| 11 | 0 | 0 | 0 | - | 0 | 100 | - |
| 12 | 0 | 0 | 100 | - | 0 | 100 | - |
| 13 | 0 | 40 | 0 | - | 0 | 100 | - |
| 14 | 0 | 0 | 0 | - | 0 | 0 | - |
| 15 | - | - | - | - | - | - | - |
| 16 | 0 | 0 | 20 | - | 0 | 100 | - |
| 17 | 0 | 0 | 0 | - | 0 | 0 | - |
| 18 | 0 | 0 | 0 | - | 0 | 100 | - |
| 19 | 0 | 0 | 0 | - | 0 | 94 | - |
| 20 | 0 | 0 | 60 | - | 0 | 100 | 85 |
| 21 | - | - | - | - | - | - | - |
| 22 | 0 | 0 | 80 | - | 0 | 100 | - |
| 23 | 20 | 0 | 100 | - | 100 | 0 | - |
| 24 | 0 | 0 | 0 | - | 0 | 0 | - |
| 25 | 0 | 0 | 0 | - | 0 | 100 | - |
| 26 | 0 | 0 | 0 | - | 0 | 100 | - |
| 27 | 0 | 0 | 0 | - | 0 | 0 | - |

| Example No. | Pesticidal Results, % Control | | | | | | |
|---|---|---|---|---|---|---|---|
| | SAW | MBB | BW | CRW[1] | GPA | MTA | NEM[1] |
| 28 | 0 | 0 | 0 | - | 0 | 0 | - |
| 29 | 0 | 0 | 40 | - | 0 | 100 | - |
| 30 | 0 | 0 | 0 | - | 0 | 100 | - |
| 31 | 0 | 0 | 0 | 0 | 0 | 0 | 0 |
| 32 | - | - | - | - | - | - | - |
| 33 | - | - | - | - | - | - | - |
| 34 | 0 | 0 | 0 | - | 0 | 100 | - |
| 35 | 0 | 0 | 0 | - | 0 | 49 | - |
| 36 | 0 | 20 | 0 | - | 0 | 100 | - |
| 37 | 0 | 0 | 0 | - | 0 | 100 | - |
| 38 | 20 | 40 | 0 | - | 0 | 100 | - |
| 39 | 0 | 0 | 0 | - | 0 | 0 | - |
| 40 | 20 | 0 | 100 | - | 0 | 0 | - |
| 41 | 0 | 0 | 0 | 0 | 0 | 0 | 0 |
| 42 | 0 | 0 | 0 | - | 0 | 0 | - |
| 43 | 0 | 0 | 0 | - | 0 | 0 | - |
| 44 | 0 | 0 | 0 | - | 0 | 0 | - |
| 45 | 0 | 0 | 0 | - | 0 | 0 | - |
| 46 | 0 | 0 | 0 | - | 0 | 0 | - |
| 47 | 0 | 0 | 0 | - | 0 | 0 | - |
| 48 | 0 | 0 | 0 | - | 0 | 0 | - |
| 49 | 0 | 0 | 0 | - | 0 | 0 | - |
| 50 | 0 | 0 | 0 | - | 0 | 0 | - |
| 51 | 0 | 0 | 0 | - | 0 | 0 | - |
| 52 | 0 | 0 | 0 | - | 0 | 0 | - |
| 53 | 0 | 0 | 0 | - | 0 | 0 | - |
| 54 | 0 | 0 | 0 | - | 0 | 0 | - |
| 55 | 0 | 0 | 0 | - | 0 | 0 | - |
| 56 | 0 | 0 | 0 | - | 0 | 0 | - |
| 57 | 0 | 0 | 0 | - | 0 | 0 | - |

| Example No. | Pesticidal Results, % Control SAW | MBB | BW | CRW[1] | GPA | MTA | NEM[1] |
|---|---|---|---|---|---|---|---|
| 58 | - | - | - | - | - | - | - |
| 59 | - | - | - | - | - | - | - |
| 60 | 0 | 0 | 0 | 0 | 0 | 0 | 0 |
| 61 | - | - | - | - | - | - | - |
| 62 | - | - | - | - | - | - | - |
| 63 | - | - | - | - | - | - | - |
| 64 | 0 | 0 | 0 | 0 | 0 | 0 | 0 |
| 65 | 0 | 0 | 0 | 0 | 0 | 0 | 0 |
| 66 | 0 | 0 | 20 | 0 | 0 | 0 | 0 |
| 67 | 0 | 0 | 20 | 0 | 0 | 0 | 0 |
| 68 | 0 | 0 | 0 | 0 | 0 | 0 | 0 |
| 69 | 0 | 0 | 0 | 0 | 0 | 0 | 0 |
| 70 | 0 | 0 | 0 | 0 | 0 | 0 | 0 |
| 71 | 0 | 0 | 0 | 0 | 0 | 100 | 0 |
| 72 | 0 | 0 | 0 | 0 | 0 | 0 | 0 |
| 73 | 0 | 0 | 0 | 0 | 0 | 0 | 0 |
| 74 | 0 | 0 | 0 | 0 | 0 | 100 | 0 |
| 75 | 0 | 0 | 20 | 0 | 0 | 0 | 0 |
| 76 | 0 | 0 | 0 | 0 | 0 | 0 | 0 |
| 77 | 100 | 0 | 20 | 0 | 0 | 100 | 0 |
| 78 | 0 | 0 | 0 | 0 | 0 | 0 | 0 |
| 79 | 0 | 0 | 0 | 0 | 0 | 0 | 0 |
| 80 | 0 | 0 | 0 | 0 | 0 | 0 | 0 |
| 81 | 0 | 0 | 0 | 0 | 0 | 0 | 0 |
| 82 | 0 | 20 | 0 | 0 | 0 | 100 | 0 |
| 83 | 0 | 0 | 20 | 0 | 0 | 100 | 0 |
| 84 | 0 | 0 | 0 | 0 | 0 | 0 | 0 |
| 85 | 0 | 0 | 20 | 0 | 0 | 100 | 5 |
| 86 | 0 | 0 | 0 | 0 | 0 | 100 | 0 |
| 87 | 0 | 0 | 0 | 0 | 0 | 100 | 0 |

| Example No. | SAW | MBB | BW | CRW[1] | GPA | MTA | NEM[1] |
|---|---|---|---|---|---|---|---|
| | Pesticidal Results, % Control | | | | | | |
| 88 | 0 | 0 | 0 | 0 | 0 | 0 | 0 |
| 89 | 0 | 0 | 0 | 0 | 0 | 0 | 0 |
| 90 | 0 | 0 | 0 | 0 | 0 | 100 | 0 |
| 91 | 0 | 0 | 0 | 0 | 0 | 0 | 0 |
| 92 | 0 | 0 | 0 | 0 | 0 | 0 | 0 |
| 93 | 0 | 0 | 0 | 0 | 0 | 0 | 0 |
| 94 | 0 | 0 | 0 | 0 | 0 | 0 | 0 |
| 95 | 0 | 0 | 0 | 0 | 0 | 0 | 0 |
| 96 | 0 | 0 | 0 | 0 | 0 | 0 | 0 |
| 97 | 0 | 0 | 0 | 0 | 0 | 0 | 0 |
| 98 | 0 | 0 | 0 | 0 | 0 | 0 | 0 |
| 99 | 0 | 0 | 0 | 0 | 0 | 0 | 0 |
| 100 | 0 | 0 | 0 | 0 | 0 | 0 | 0 |
| 101 | 0 | 0 | 0 | 0 | 0 | 0 | 0 |
| 102 | 0 | 0 | 0 | 0 | 0 | 100 | 0 |
| 103 | 0 | 0 | 0 | 0 | 0 | 44 | 0 |
| 104 | 0 | 0 | 0 | 0 | 0 | 0 | 0 |
| 105 | 0 | 0 | 0 | 0 | 0 | 0 | 0 |
| 106 | 0 | 0 | 20 | 0 | 0 | 0 | 0 |
| 107 | 0 | 0 | 0 | 0 | 0 | 0 | 0 |
| 108 | 0 | 0 | 0 | 0 | 0 | 0 | 0 |
| 109 | 0 | 20 | 0 | 0 | 0 | 0 | 0 |
| 110 | 0 | 0 | 0 | 0 | 0 | 0 | 0 |
| 111 | 0 | 0 | 0 | 0 | 0 | 0 | 0 |
| 112 | 0 | 0 | 0 | 0 | 0 | 0 | 0 |
| 113 | 0 | 0 | 0 | 0 | 0 | 0 | 0 |
| 114 | 0 | 0 | 0 | 0 | 0 | 0 | 0 |
| 115 | 0 | 0 | 0 | 0 | 0 | 0 | 0 |
| 116 | 0 | 0 | 0 | 0 | 0 | 0 | 0 |
| 117 | 0 | 0 | 0 | 0 | 0 | 0 | 0 |

| Example No. | Pesticidal Results, % Control | | | | | | |
|---|---|---|---|---|---|---|---|
| | SAW | MBB | BW | CRW[1] | GPA | MTA | NEM[1] |
| 118 | 0 | 0 | 0 | 0 | 0 | 0 | 0 |
| 119 | 0 | 0 | 0 | 0 | 0 | 0 | 0 |

[1] Tested at 150 ppm.
[2] No data recorded.

Generally, control of a microorganism is achieved in accordance with the invention by contacting the organism directly or by contacting a locus subject to contamination by microorganisms with a polyhaloketoxime in an amount which is effective to control said organism. Any of the techniques known in the art can be employed to disseminate the compounds of this invention in a manner so as to achieve the desired contact. Spraying and fumigating are typical of such techniques.

The compounds of this invention can be readily utilized as bactericides, fungicides and pesticides or combinations thereof in any locus, such as, for example, paper pulp processes, aqueous polymer dispersions, water-based paints, seed treatment applications and the like. In addition, these compounds and compositions containing them can function as, for example, fabric or leather preservatives, wood preservatives, cosmetic preservatives, soap additives, sanitizing agents, such as in laundry soaps and detergents, and preservatives for metal working compounds, such as emulsifiable cutting oils, preservatives for fuels, fiber spin finish biocides and the like.

In general, a locus subject to contamination by microorganisms can be protected in accordance with this invention by incorporating into the locus a polyhaloketoxime in an amount which is effective to control said microorganisms. The term "contamination" is meant to include any attack by microorganisms which leads to a chemical or physical breakdown or disintegration of the locus as well as the proliferation of the microorganisms within the locus without an accompanying deleterious effect. The exact amount of polyhaloketoxime required will, of course, vary with the medium being controlled, the particular polyhaloketoxime or compositions containing the polyhaloketoxime being employed and other factors. Typically, in a liquid medium, excellent control is obtained when the polyhaloketoximes are incorporated in the range of from about 0.01 to about 10,000 parts per million (ppm) or up to 95% based on the weight of the composition. A range of from about 0.05 to about 2,500 ppm is preferred.

The term "control" or "combat" as employed in this application is to be construed as the effect of any means which adversely affects the existence or growth of any living organism or microorganism. This effect may comprise a complete killing action, eradication, arresting in growth, inhibition, reduction in number or any combination thereof.

The polyhaloketoximes of this invention are useful as agricultural fungicides. As such, they are particularly valuable when formulated in a fungicidal composition. Such compositions normally comprise an agronomically acceptable carrier and a polyhaloketoxime or mixture of polyhaloketoximes as the active agent.

Where necessary or desirable, surfactants or other additives may be incorporated to give uniformly formulated mixtures. By "agronomically acceptable carrier" is meant any substance which can be utilized to dissolve, dispense or diffuse the chemical incorporated therein without impairing the effectiveness of the toxic agent and which does no permanent damage to such environment as soil, equipment and agronomic crops.

For use as agricultural fungicides, the compounds of this invention are usually taken up on an agronomically acceptable carrier or formulated so as to render them suitable for subsequent dissemination. For example, the polyhaloketoximes can be formulated as wettable powders, emulsion concentrates, dusts, granular formulations, aerosols or flowable emulsifiable concentrates. In such formulations, the polyhaloketoximes are extended with a liquid or solid carrier and, when desired, suitable surfactants are likewise incorporated.

For use as pesticides, the compounds of this invention can be used as solutions in organic solvents or formulations. For example, they can be formulated as wettable powders, emulsifiable concentrates, dusts, granular formulations or flowable emulsifiable concentrates. In such formulations, the polyhaloketoximes are extended with an agronomically acceptable liquid or solid carrier and, when desired, suitable surfactants are incorporated. Surfactants commonly used in the art can be found in the John W. McCutcheon, Inc. publication "Detergents and Emulsifiers Annual".

Compounds of this invention can be dissolved in a water-miscible liquid, such as ethanol, isopropanol,

acetone, and the like. Such solutions are easily extended with water.

The polyhaloketoximes can be taken up on or mixed with a finely particled solid carrier as, for example, clays, inorganic silicates, carbonates, and silicas. Organic carriers can also be employed. Dust concentrates are commonly made wherein polyhaloketoximes are present in the range of from about 20% to about 80% by weight. For ultimate applications, these concentrates are normally extended with additional solids from about 1% to about 20% by weight.

Wettable powder formulations are made by incorporating the compounds of this invention in an inert, finely divided solid carrier along with a surfactant which may be one or more emulsifying, wetting, dispersing or spreading agents or blend of these. The polyhaloketoximes are usually present in the range of from about 10% to about 80% by weight and the surfactants are from about 0.5% to about 10% by weight. Commonly used emulsifying and wetting agents include polyoxyethylated derivatives of alkylphenols, fatty alcohols, fatty acids, and alkylamines, alkylarene sulfonates and dialkyl sulfosuccinates. Spreading agents include such materials as glycerol mannitan laurate and a condensate of polyglycerol and oleic acid modified with phthalic anhydride. Dispersing agents include such materials as the sodium salt of the copolymer of maleic anhydride and an olefin such as diisobutylene, sodium lignin sulfonate and sodium formaldehydenaphthalene sulfonates.

One convenient method for preparing a solid formulation is to impregnate the polyhaloketoxime toxicant onto the solid carrier by means of a volatile

solvent, such as acetone. In this manner adjuvants, such as activators, adhesives, plant nutrients, synergists and various surfactants, can also be incorporated.

Emulsifiable concentrate formulations can be prepared by dissolving the polyhaloketoximes of this invention in an agronomically acceptable organic solvent and adding a solvent-soluble emulsifying agent. Suitable solvents are usually water-immiscible and may be found in the hydrocarbon, chlorinated hydrocarbon, ketone, ester, alcohol and amide classes of organic solvents. Mixtures of solvents are commonly employed. The surfactants useful as emulsifying agents may constitute from about 0.5% to about 10% by weight of the emulsifiable concentrate and may be anionic, cationic or non-ionic in character. Anionic surfactnts include alcohol sulfates or sulfonates, alkylarene sulfonates and sulfosuccinates. Cationic surfactants include fatty acid alkylamine salts and fatty acid alkyl quaternaries. Nonionic emulsifying agents include alkylene oxide adducts of alkylphenols, fatty alcohols, mercaptans and fatty acids. The concentration of the active ingredients may vary from about 10% to about 80%, preferably in the range of from about 25 to about 50%.

For use as a fungicidal agent, these compounds should be applied in an effective amount sufficient to exert the desired biocidal activity by techniques well-known in the art. Usually, this will involve the application of the polyhaloketoximes to the locus to be protected in an effective amount when incorporated in an agronomically acceptable carrier. However, in certain situations, it may be desirable and advantageous to apply the compounds directly onto the

locus to be protected without the benefit of any substantial amount of carrier. This is a particularly effective method when the physical nature of the polyhaloketoxime is such as to permit what is known as "low-volume" application; that is, when the compounds are in liquid form or substantially soluble in higher boiling solvents.

The application rate will, of course, vary depending upon the purpose for such application, the polyhaloketoxime being utilized, the frequency of dissemination and the like.

For use as pesticidal agents, these compounds should be applied in an effective amount sufficient to exert the desired pesticidal activity by techniques well known in the art. Usually, this will involve the application of the polyhaloketoxime to the loci to be protected from or freed of pests in an effective amount when incorporated in an agronomically acceptable carrier. However, in certain situations, it may be desirable and advantageous to apply the compounds directly onto the loci to be protected from or freed of pests without the benefit of any substantial amount of carrier. This is a particularly effective method when the physical nature of the toxicants is such as to permit what is known as "low-volume" application, that is, when the compounds are in liquid form or substantially soluble in higher boiling solvents.

The application rate will, of course, vary depending upon the purpose of such application, the polyhaloketoxime being utilized, the frequency of dissemination and the like.

For use as agricultural bactericides and fungicides, dilute sprays can be applied at concentrations of from about 0.05 to about 20 pounds of

the active polyhaloketoximes ingredient per 100 gallon of spray. They are usually applied at from about 0.1 to about 10 pounds per 100 gallons and preferably between about 0.125 to about 5 pounds per 100 gallons. In more concentrated sprays, the active ingredient is increased by a factor of 2 to 12. With dilute sprays, applications are usually made to the plants until run-off is achieved; whereas, with more concentrated or low-volume sprays, the materials are applied as mists.

For use as insecticides and acaricides, dilute sprays can be applied at concentrations of from about 0.01 to about 20 pounds of the polyhaloketoxime ingredient per 100 gallons of spray. They are usually applied at from about 0.1 to about 5 pounds per 100 gallons. In more concentrated sprays, the active ingredient is increased by a factor of from about 2 to about 12. With dilute sprays, applications are usually made to the plants until run-off is achieved, whereas with more concentrated low-volume sprays the materials are applied as mists.

As a fungicidal seed protectant, the amount of toxicant coated on the seed is usually at a dosage rate of from about 0.1 to about 20 ounces per hundred pounds of seed. As a soil fungicide the chemical can be incorporated in the soil or applied to the surface usually at a rate of from about 0.1 to about 50 lbs. per acre. As a foliar fungicide, the toxicant is usually applied to growing plants at a rate of from about 0.25 to about 10 lbs. per acre.

For use as a nematocide, systemic agent, or as a soil insecticide, the polyhaloketoximes can be applied as a solid formulation, preferably a granular formulation or as a diluted liquid preparation, by

broadcasting, side-dressing, soil incorporation or seed treatment.

The composition can also be added to transplant water or employed as dips or soaks for vegetative parts employed in propagation, such as seeds, tubers, roots, seedlings, etc., so as to disinfect and/or provide residual protection from nematodes, soil insects (and mites) and via systemic uptake, foliar pests. The application rate can be from about 0.5 to about 50 pounds per acre; however, higher rates can also be used. The preferred rate is from about 1 to about 25 pounds per acre. For soil incorporation, the compounds of this invention can be mixed with the soil at a rate of about 1 to about 100 ppm of active ingredient.

The compounds of this invention may be utilized as the sole biocidal agents or they may be employed in conjunction with other fungicides, bactericides, algaecides, slimicides, insecticides, miticides, or with other comparable pesticides.

There should also be mentioned as useful, those compounds of this invention which comprise each and every one of the compounds of the invention listed hereinbefore by name or formula when modified by the exchange of one or more of the substituents for one or more of the different substituents present in any other one of said compounds or stated as preferred substituents hereinbefore.

CLAIMS

1. A compound of the formula

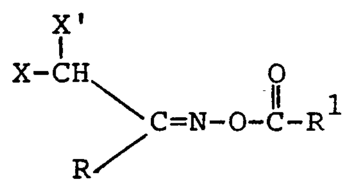

(I)

wherein

X and X' are the same or different halogen atoms;

R is methyl or halomethyl; and

$R^1$ is $(C_1-C_{12})$alkyl optionally substituted with one or more halogen, $(C_1-C_4)$alkoxy, cyano or nitro;

$(C_3-C_6)$cycloalkyl;

straight or branched chain $(C_1-C_4)$alkoxy optionally substituted with one or more halogen;

an amino group of the formula $NHR^2$ where $R^2$ is hydrogen, straight or branched chain $(C_1-C_4)$alkyl optionally substituted with one or more of the same or different halogen, $(C_3-C_6)$cycloalkyl, straight or branched chain $(C_2-C_6)$alkenyl, or aryl;

a thio group of the formula $SR^3$ where $R^3$ is straight or branched chain $(C_1-C_4)$alkyl optionally substituted with one or more of the same or different halogen, or aryl;

$(C_2-C_6)$alkenyl optionally substituted with one or more halogen;

aryl optionally substituted with one or more of the same or different halogen, cyano, nitro, $(C_1-C_4)$alkyl, $(C_1-C_4)$alkoxy, or $(C_1-C_4)$haloalkyl,

a heterocyclic group;

phenalkyl where the alkyl moiety contains one to four carbon atoms and the phenyl ring is optionally substituted with one or more of the same or different halogen, cyano, nitro, $(C_1-C_4)$alkyl, $(C_1-C_4)$alkoxy, or $(C_1-C_4)$-haloalkyl;

phenalkenyl where the alkenyl moiety contains two to five carbon atoms and the phenyl ring is optionally substituted with one or more of the same or different halogen, cyano, nitro, $(C_1-C_4)$alkyl, $(C_1-C_4)$alkoxy, or $(C_1-C_4)$-haloalkyl;

phenoxy optionally substituted with one or more of the same or different halogen, cyano, nitro, $(C_1-C_4)$alkyl, $(C_1-C_4)$alkoxy, or $(C_1-C_4)$haloalkyl; or

benzyloxy where the phenyl ring is optionally substituted with one or more of the same or different halogen, cyano, nitro, $(C_1-C_4)$-alkyl, $(C_1-C_4)$alkoxy, or $(C_1-C_4)$haloalkyl.

2. A compound according to claim 1 wherein

X and X' are the same or different halogen selected from chloro, bromo or fluoro;

$R^1$ is $(C_1-C_8)$alkyl optionally substituted with one or more of the same or different halogen;

$(C_3-C_6)$cycloalkyl;

straight or branched chain $(C_1-C_4)$alkoxy optionally substituted with one or more halogen;

an amino group of the formula $NHR^2$ where $R^2$ is hydrogen, straight or branched chain $(C_1-C_4)$alkyl, or aryl;

a thio group of the formula $SR^3$ where $R^3$ is straight or branched chain $(C_1-C_4)$alkyl;

$(C_2-C_6)$alkenyl, optionally substituted with one or more halogen;

aryl, optionally substituted with one or more of the same or different halogen, cyano, nitro, $(C_1-C_4)$alkyl, $(C_1-C_4)$alkoxy, or $(C_1-C_4)$haloalkyl;

furyl;

phenalkyl where the alkyl moiety contains one to four carbon atoms; or

phenalkenyl where the alkenyl moiety contains two to five carbon atoms and the phenyl ring is optionally substituted with one or more of the same or different halogen, cyano, nitro, $(C_1-C_4)$alkyl, $(C_1-C_4)$alkoxy, or $(C_1-C_4)$haloalkyl.

3. A compound according to claim 2 wherein
X and X' are chloro;
R is methyl, chloromethyl or dichloromethyl; and
$R^1$ is $(C_1-C_4)$alkyl optionally substituted with one to three of the same or different halogen;

$(C_3-C_6)$cycloalkyl, preferably cyclohexyl;

$(C_1-C_4)$alkoxy;

$(C_2-C_5)$alkenyl;

an amino group of the formula $NHR^2$ where $R^2$ is $(C_1-C_4)$alkyl or phenyl;

a phenyl optionally substituted with one to three of the same or different halogen, nitro, $(C_1-C_4)$alkyl or $(C_1-C_4)$alkoxy;

furyl;

phenalkyl where the alkyl moiety contains one to four carbon atoms; or

phenalkenyl where the alkenyl moiety contains two to five carbon atoms.

4. A compound according to claim 1 wherein X and X' are chloro; and R is methyl; and $R^1$ is methyl, or ethyl, or methoxy, or ethoxy, or 4-chlorophenyl, or phenyl, or 2-furyl, or 3,4-dichlorophenyl, or 4-fluorophenyl, or 4-methylphenyl, or *n*-propyl, or isopropenyl, or 1-isobutenyl, or 2-chloroethyl, or 4-nitrophenyl, or 1-chloroethyl, or styryl, or anilino, or 3-chlorophenyl, or methylamino; or R is dichloromethyl and $R^1$ is phenyl, or 4-methylphenyl, or styryl, or 4-chlorophenyl, or 3-chlorophenyl, or 3-methoxyphenyl, or 4-methoxyphenyl, or 3-methylphenyl, or 4-fluorophenyl; or R is chloromethyl and $R^1$ is 4-chlorophenyl, or ethyl, or methoxy, or isopropenyl, or cyclohexyl, or benzyl, or 2-phenylethyl, or 2-chloroethyl, or 4-fluorophenyl, or 4-bromophenyl, or 2-chlorophenyl, or 3-chlorophenyl, or 3,5-dichlorophenyl, or phenyl, or 4-methylphenyl, or 4-methoxyphenyl, or 1,3-pentadienyl, or 1-isobutenyl, or ethoxy, or 1-propenyl, or 3-trifluoromethylphenyl.

5. A process for the preparation of a compound according to claim 1 comprising reacting, in the presence of solvent, a compound of the formula

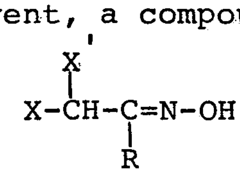

(II)

with either (when $R^1$ is other than $NHR^2$)

a compound of the formula

$$Z-\overset{\overset{O}{\|}}{C}-R^1 \qquad \text{(III)}$$

in the presence of a suitable base,

or (when $R^1$ is $NHR^2$)

a compound of the formula

$$O=C=N-R^2; \qquad \text{(IV)}$$

wherein X, X', R, $R^1$ and $R^2$ are as defined in Claim 1 and Z is a halo atom.

6. A process as claimed in Claim 5 using the compound of formula (III) in an amount of 1 to 1.2 mole and base in an amount of 1 to 1.2 mole per mole of compound of formula (II) and carried out at a temperature of 0 to 80°C or using the compound of formula (IV) in an amount of 1 to 4 moles per mole of compound of formula (II) and carried out at a temperature of 0 to 110°C.

7. A fungicidal, bactericidal or pesticidal composition comprising as an active ingredient a compound according to any one of Claims 1 to 4 or made by a process according to Claim 5 or 6 and an environmentally acceptable diluent or carrier for the active ingredient.

8. A composition according to Claim 7 wherein the diluent or carrier is an agronomically acceptable diluent or carrier.

9. A method for controlling fungi, bacteria or pests which comprises applying directly to fungi, bacteria or pests or to a loci to be freed or protected

from attack by fungi, bacteria or pests, a fungicidally, bactericidally or pesticidally effective amount of a compound according to any one of Claims 1 to 4 or made by a process according to Claim 5 or 6, optionally in a composition according to Claim 7 or 8.

CLAIMS: AT

1. A fungicidal, bactericidal or pesticidal composition comprising a compound of the formula

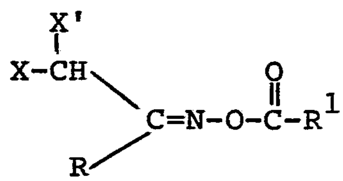

(I)

wherein

X and X' are the same or different halogen atoms;

R is methyl or halomethyl; and

$R^1$ is $(C_1-C_{12})$alkyl optionally substituted with one or more halogen, $(C_1-C_4)$alkoxy, cyano or nitro;

$(C_3-C_6)$cycloalkyl;

straight or branched chain $(C_1-C_4)$alkoxy optionally substituted with one or more halogen;

an amino group of the formula $NHR^2$ where $R^2$ is hydrogen, straight or branched chain $(C_1-C_4)$alkyl optionally substituted with one or more of the same or different halogen, $(C_3-C_6)$cycloalkyl, straight or branched chain $(C_2-C_6)$alkenyl, or aryl;

a thio group of the formula $SR^3$ where $R^3$ is straight or branched chain $(C_1-C_4)$alkyl optionally substituted with one or more of the same or different halogen, or aryl;

($C_2$-$C_6$)alkenyl optionally substituted with one or more halogen;

aryl optionally substituted with one or more of the same or different halogen, cyano, nitro, ($C_1$-$C_4$)alkyl, ($C_1$-$C_4$)alkoxy, or ($C_1$-$C_4$)haloalkyl,

a heterocyclic group;

phenalkyl where the alkyl moiety contains one to four carbon atoms and the phenyl ring is optionally substituted with one or more of the same or different halogen, cyano, nitro, ($C_1$-$C_4$)alkyl, ($C_1$-$C_4$)alkoxy, or ($C_1$-$C_4$)-haloalkyl;

phenalkenyl where the alkenyl moiety contains two to five carbon atoms and the phenyl ring is optionally substituted with one or more of the same or different halogen, cyano, nitro, ($C_1$-$C_4$)alkyl, ($C_1$-$C_4$)alkoxy, or ($C_1$-$C_4$)-haloalkyl;

phenoxy optionally substituted with one or more of the same or different halogen, cyano, nitro, ($C_1$-$C_4$)alkyl, ($C_1$-$C_4$)alkoxy, or ($C_1$-$C_4$)haloalkyl; or

benzyloxy where the phenyl ring is optionally substituted with one or more of the same or different halogen, cyano, nitro, ($C_1$-$C_4$)-alkyl, ($C_1$-$C_4$)alkoxy, or ($C_1$-$C_4$)haloalkyl;

and an environmentally acceptable carrier or diluent therefor.

2. A composition according to claim 1 wherein

X and X' are the same or different halogen selected from chloro, bromo or fluoro;

$R^1$ is $(C_1-C_8)$alkyl optionally substituted with one or more of the same or different halogen;

$(C_3-C_6)$cycloalkyl;

straight or branched chain $(C_1-C_4)$alkoxy optionally substituted with one or more halogen;

an amino group of the formula $NHR^2$ where $R^2$ is hydrogen, straight or branched chain $(C_1-C_4)$alkyl, or aryl;

a thio group of the formula $SR^3$ where $R^3$ is straight or branched chain $(C_1-C_4)$alkyl;

$(C_2-C_6)$alkenyl, optionally substituted with one or more halogen;

aryl, optionally substituted with one or more of the same or different halogen, cyano, nitro, $(C_1-C_4)$alkyl, $(C_1-C_4)$alkoxy, or $(C_1-C_4)$haloalkyl;

furyl;

phenalkyl where the alkyl moiety contains one to four carbon atoms; or

phenalkenyl where the alkenyl moiety contains two to five carbon atoms and the phenyl ring is optionally substituted with one or more of the same or different halogen, cyano, nitro, $(C_1-C_4)$alkyl, $(C_1-C_4)$alkoxy, or $(C_1-C_4)$haloalkyl.

3. A composition according to claim 2 wherein

X and X' are chloro;

R is methyl, chloromethyl or dichloromethyl; and

$R^1$ is $(C_1-C_4)$alkyl optionally substituted with one to three of the same or different halogen;

$(C_3-C_6)$cycloalkyl, preferably cyclohexyl;

$(C_1-C_4)$alkoxy;

$(C_2-C_5)$alkenyl;

an amino group of the formula $NHR^2$ where $R^2$ is $(C_1-C_4)$alkyl or phenyl;

a phenyl optionally substituted with one to three of the same or different halogen, nitro, $(C_1-C_4)$alkyl or $(C_1-C_4)$alkoxy;

furyl;

phenalkyl where the alkyl moiety contains one to four carbon atoms; or

phenalkenyl where the alkenyl moiety contains two to five carbon atoms.

4. A composition according to claim 1 wherein
X and X' are chloro; and
R is methyl; and
$R^1$ is methyl, or ethyl, or methoxy, or ethoxy, or 4-chlorophenyl, or phenyl, or 2-furyl, or 3,4-dichlorophenyl, or 4-fluorophenyl, or 4-methylphenyl, or _n_-propyl, or isopropenyl, or 1-isobutenyl, or 2-chloroethyl, or 4-nitrophenyl, or 1-chloroethyl, or styryl, or anilino, or 3-chlorophenyl, or methylamino; or
R is dichloromethyl and
$R^1$ is phenyl, or 4-methylphenyl, or styryl, or 4-chlorophenyl, or 3-chlorophenyl, or 3-methoxyphenyl, or 4-methoxyphenyl, or 3-methylphenyl, or 4-fluorophenyl; or
R is chloromethyl and
$R^1$ is 4-chlorophenyl, or ethyl, or methoxy, or isopropenyl, or cyclohexyl, or benzyl, or 2-phenylethyl, or 2-chloroethyl, or 4-fluorophenyl, or 4-bromophenyl, or 2-chlorophenyl, or 3-chlorophenyl, or 3,5-dichlorophenyl, or phenyl, or 4-methylphenyl, or 4-methoxyphenyl, or 1,3-pentadienyl, or 1-isobutenyl, or ethoxy, or 1-propenyl, or 3-trifluoromethylphenyl.

5. A composition according to any preceding claim wherein the diluent or carrier is an agronomically acceptable diluent or carrier.

6. A process for the preparation of a compound according to formula (I) as defined in claim 1 comprising reacting, in the presence of solvent, a compound of the formula

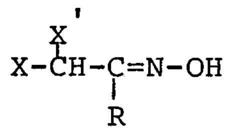 (II)

with either (when $R^1$ is other than $NHR^2$)

a compound of the formula

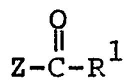 (III)

in the presence of a suitable base,

or (when $R^1$ is $NHR^2$)

a compound of the formula

$O=C=N-R^2$; (IV)

wherein X, X', R, $R^1$ and $R^2$ are as defined in Claim 1 and Z is a halo atom.

7. A process as claimed in Claim 6 using the compound of formula (III) in an amount of 1 to 1.2 mole and base in an amount of 1 to 1.2 mole per mole of compound of formula (II) and carried out at a temperature of 0 to 80°C or using the compound of formula (IV) in an amount of 1 to 4 moles per mole of compound of formula (II) and carried out at a temperature of 0 to 110°C.

8. A method for controlling fungi, bacteria or pests which comprises applying directly to fungi, bacteria or pests or to a loci to be freed or protected from attack by fungi, bacteria or pests, a fungicidally, bactericidally or pesticidally effective amount of a compound of formula (I) as defined in any one of Claims 1 to 4 or made by a process according to Claim 6 or 7, optionally in a composition according to any of Claims 1 to 5.